(19) Europäisches Patentamt
European Patent Office
Office européen des brevets

(11) EP 3 988 173 A1

(12) **EUROPEAN PATENT APPLICATION**
published in accordance with Art. 153(4) EPC

(43) Date of publication:
**27.04.2022 Bulletin 2022/17**

(21) Application number: **20825777.4**

(22) Date of filing: **17.06.2020**

(51) International Patent Classification (IPC):
***A61P 35/00*** *(2006.01)* ***C07H 19/14*** *(2006.01)*
***A61K 31/7064*** *(2006.01)*

(52) Cooperative Patent Classification (CPC):
**A61K 31/7064; A61P 35/00; C07H 19/14**

(86) International application number:
**PCT/JP2020/023660**

(87) International publication number:
**WO 2020/255978 (24.12.2020 Gazette 2020/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.06.2019 JP 2019113172**
**29.08.2019 JP 2019157375**

(71) Applicant: **Taiho Pharmaceutical Co., Ltd.**
**Chiyoda-ku**
**Tokyo 101-8444 (JP)**

(72) Inventors:
- **MIYAKOSHI, Hitoshi**
**Tsukuba-shi, Ibaraki 300-2611 (JP)**
- **TANAKA, Nozomu**
**Tsukuba-shi, Ibaraki 300-2611 (JP)**
- **KOBAYAKAWA, Yu**
**Tsukuba-shi, Ibaraki 300-2611 (JP)**

(74) Representative: **Schiener, Jens**
**Wächtershäuser & Hartz**
**Patentanwaltspartnerschaft mbB**
**Weinstraße 8**
**80333 München (DE)**

(54) **NOVEL PHOSPHATE ESTER COMPOUND HAVING PYRROLOPYRIMIDINE SKELETON OR PHARMACEUTICALLY ACCEPTABLE SALT THEREOF**

(57) There is provided a novel phosphate ester compound having a pyrrolopyrimidine skeleton having an excellent antitumor effect or a pharmaceutically acceptable salt thereof, and a method for preventing and/or treating a tumor in combination with an alkylating agent and/or a radiation therapy. According to one aspect of the present invention, there is provided a compound represented by the following general formula (1) or a pharmaceutically acceptable salt thereof, and a method for preventing and/or treating a tumor in combination with an alkylating agent and/or a radiation therapy.

EP 3 988 173 A1

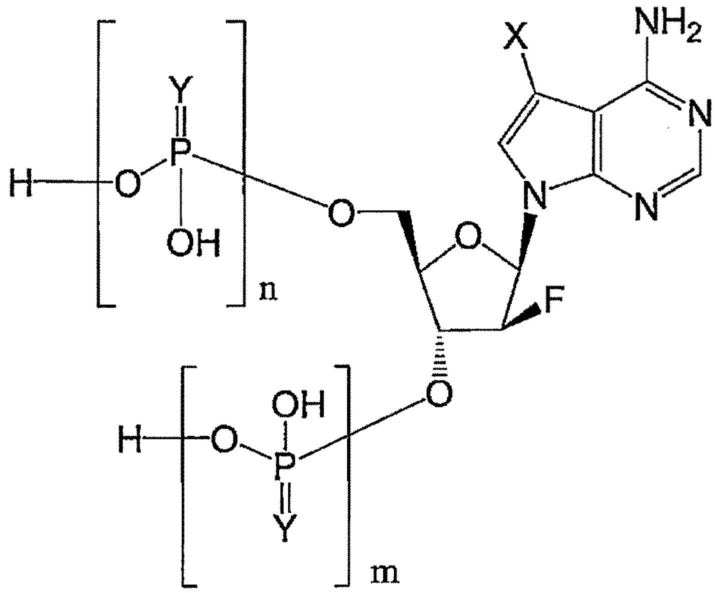

(1)

## Description

### Technical Field

The present invention relates to a novel phosphate ester compound having a pyrrolopyrimidine skeleton having an excellent antitumor effect, or a pharmaceutically acceptable salt thereof. The present invention also relates to combination use of a novel phosphate ester compound having a pyrrolopyrimidine skeleton or a pharmaceutically acceptable salt thereof and an alkylating agent and/or a radiation therapy.

### Background Art

Tumors characterized by abnormal proliferation of cells are intractable diseases for which effective treatments are still desired. For proliferation of tumor cells, biosynthesis of nucleic acids is essential. The compounds which mimic molecules involved in biosynthesis of nucleic acids have been energetically developed as nucleic acid antimetabolites disturbing nucleic acid metabolism of tumors.

However, even if compounds having a highly effective nucleic acid antimetabolic action are found out, most of the compounds may have problems in view of toxicity or pharmacokinetics and cannot be used as clinically useful medical agents. To overcome the problems and maximize the efficacy which these compounds inherently have, the compounds are sometimes converted into prodrugs. If a compound having an antitumor effect is converted into a prodrug, it is expected that the pharmacokinetics of the compound and selectivity of action thereof on a cancer tissue can be improved. However, despite such expectations, it is not easy to convert a compound having a highly effective nucleic acid antimetabolic action into a prodrug serving as a clinically useful medical agent.

As a compound having a pyrrolopyrimidine skeleton, deoxyribonucleoside derivatives having a 4-amino-5-halogeno-7H-pyrrolo[2,3-d]pyrimidine skeleton (Non Patent Literatures 1, 2, 3, 4) and a deoxyribonucleoside derivative having an iodine atom at the 5-position (Patent Literature 1) have been reported. However, there are no descriptions suggesting nucleotide derivatives corresponding to these nucleoside derivatives.

Deoxyribonucleotide derivatives having a 4-amino-5-halogeno-7H-pyrrolo[2,3-d]pyrimidine skeleton have been reported (Patent Literatures 2, 3). Also, a derivative having a fluorine atom at the 5-position of a deoxyribonucleotide derivative has been reported (Patent Literature 3). Furthermore, a deoxyribonucleotide derivative having a 4-amino-7H-pyrrolo[2,3-d]pyrimidine skeleton has been reported (Patent Literature 1). However, regarding these nucleotides, the literatures have no descriptions suggesting prodrugs of the corresponding nucleoside derivatives.

Moreover, a ribonucleotide derivative having a phosphate at the 3'-position has been reported (Non Patent Literature 5). However, regarding these nucleotide derivatives, the literatures neither describe a compound having a halogen atom at the 5-position of a pyrrolo[2,3-d]pyrimidine skeleton nor suggest prodrugs of the corresponding nucleoside derivatives.

### Citation List

#### Patent Literature

Patent Literature 1: WO 2013/009735
Patent Literature 2: WO 2014/124430
Patent Literature 3: WO 2017/165489


#### Non Patent Literature

Non Patent Literature 1: Li et al., Acta Cryst. 2014, E70, o120
Non Patent Literature 2: Perlikova et al., ChemMedChem, 2013, 8, 832-846
Non Patent Literature 3: Naus et al., Bioorg. Med. Chem, 20, 2012, 5202-5214
Non Patent Literature 4: Brown et al., J Med Chem. 2016 July 28; 59(14): 6860-6877
Non Patent Literature 5: Ikehara et al., Chemical & Pharmaceutical Bulletin 1966, 14(12), 1338-46

Summary of Invention

Technical Problem

The present invention provides a compound having a pyrimidine skeleton, which has more excellent safety than existing compounds having a pyrimidine skeleton and exerts a high antitumor effect. The present invention also provides a combination therapy of the compound having such a pyrimidine skeleton and an alkylating agent and/or a radiation therapy.

Solution to Problem

The present inventors found deoxyribonucleotide derivatives having a pyrrolo[2,3-d]pyrimidine skeleton represented by general formula (1). These compounds have a predetermined halogen atom at the 5-position and a phosphate at the 3'-position or the 5'-position.

More specifically, an embodiment of the present invention includes the followings.

[1] A compound represented by the following general formula (1):

[Formula 1]

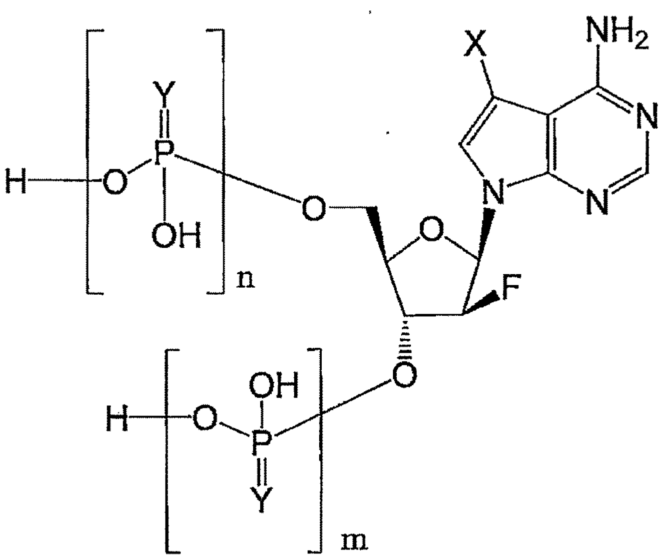

(1)

wherein

X represents a chlorine atom, a bromine atom or an iodine atom,
Ys, which may be the same or different, each represent an oxygen atom or a sulfur atom,
m represents an integer of 0 or 1,
n represents an integer of 0 or 1, and
m + n = 1,

or a pharmaceutically acceptable salt thereof.

[2] The compound or a pharmaceutically acceptable salt thereof according to [1], wherein X represents a bromine atom or an iodine atom.

[3] The compound or a pharmaceutically acceptable salt thereof according to [2], wherein X represents an iodine atom.

[4] The compound or a pharmaceutically acceptable salt thereof according to [3], wherein Y represents an oxygen atom.

[5] The compound or a pharmaceutically acceptable salt thereof according to [4], wherein m = 0 and n = 1.

[6] The compound or a pharmaceutically acceptable salt thereof according to [1], wherein X represents a bromine atom or an iodine atom; Y represents an oxygen atom; m = 0; and n = 1.

[7] A compound selected from the group consisting of:

(1) ((2R,3R,4S,5R)-5-(4-amino-5-bromo-7H-pyrrolo[2,3-d]pyrimidin-7-yl)-4-fluoro-3-hydroxytetrahydrofuran-2-yl)methyl dihydrogen phosphate;
(2) ((2R,3R,4S,5R)-5-(4-amino-5-iodo-7H-pyrrolo[2,3-d]pyrimidin-7-yl)-4-fluoro-3-hydroxytetrahydrofuran-2-yl)methyl dihydrogen phosphate;
(3) O-(((2R,3R,4S,5R)-5-(4-amino-5-iodo-7H-pyrrolo[2,3-d]pyrimidin-7-yl)-4-fluoro-3-hydroxytetrahydrofuran-2-yl)methyl) O,O-dihydrogen phosphorothioate;
(4) (2R,3R,4S,5R)-5-(4-amino-5-iodo-7H-pyrrolo[2,3-d]pyrimidin-7-yl)-4-fluoro-2-(hydroxymethyl) tetrahydrofuran-3-yl dihydrogen phosphate; and
(5) (2R,3R,4S,5R)-5-(4-amino-5-chloro-7H-pyrrolo[2,3-d]pyrimidin-7-yl)-4-fluoro-3-hydroxytetrahydrofuran-2-yl)methyl dihydrogen phosphate or a pharmaceutically acceptable salt thereof.

[8] An antitumor agent comprising the compound or a pharmaceutically acceptable salt thereof according to any one of [1] to [7], as an active ingredient.
[9] A pharmaceutical composition comprising the compound or a pharmaceutically acceptable salt thereof according to any one of [1] to [7], and a pharmaceutically acceptable carrier.
[10] The antitumor agent according to [8] or the pharmaceutical composition according to [9], formulated as an oral preparation or an injection.
[11] A method for preventing and/or treating a tumor, comprising administering the compound or a pharmaceutically acceptable salt thereof according to any one of [1] to [7].
[12] A method for preventing and/or treating a tumor, comprising administering the compound or a pharmaceutically acceptable salt thereof according to any one of [1] to [7], formulated as an oral preparation or an injection, to a subject in need thereof.
[13] The compound or a pharmaceutically acceptable salt thereof according to any one of [1] to [7] for use as a pharmaceutical composition.
[14] The compound or a pharmaceutically acceptable salt thereof according to any one of [1] to [7], for preventing and/or treating a tumor.
[15] The compound or a pharmaceutically acceptable salt thereof according to any one of [1] to [7], formulated as an oral preparation or an injection, for use in prevention and/or treatment of a tumor.
[16] Use of the compound or a pharmaceutically acceptable salt thereof according to any one of [1] to [7], for producing an antitumor agent.
[17] Use of the compound or a pharmaceutically acceptable salt thereof according to any one of [1] to [7], for producing a pharmaceutical composition.
[18] Use of the compound or a pharmaceutically acceptable salt thereof according to any one of [1] to [7], for producing an antitumor agent, formulated as an oral preparation or an injection.
[19] The antitumor agent according to [8], the method for preventing and/or treating a tumor according to [11] or [12], the compound or a pharmaceutically acceptable salt thereof according to any one of [13] to [15] or the use according to any one of [16] to [18], wherein the tumor is selected from the group consisting of head and neck cancer (e.g., oral cancer, pharyngeal cancer, laryngeal cancer, nasal cancer, sinus cancer, salivary gland cancer, thyroid cancer), gastrointestinal cancer (e.g., esophageal cancer, stomach cancer, duodenal cancer, liver cancer, biliary tract cancer (e.g., gall bladder/bile duct cancer), pancreatic cancer, colorectal cancer (e.g., colon cancer, rectal cancer)), lung cancer (e.g., non-small cell lung cancer, small cell lung cancer, mesothelioma), breast cancer, genital cancer (e.g., ovarian cancer, uterine cancer (e.g., cervical cancer, endometrial cancer)), urinary cancer (e.g., kidney cancer, bladder cancer, prostate cancer, a testicular tumor), a hematopoietic organ tumor (e.g., leukemia, malignant lymphoma, multiple myeloma), a bone/soft tissue tumor, skin cancer and a brain tumor.
[20] The antitumor agent, method for preventing and/or treating a tumor, compound or a pharmaceutically acceptable salt thereof or use according to [19], wherein the tumor is a brain tumor.
[21] A compound represented by the following general formula (2):

[Formula 2]

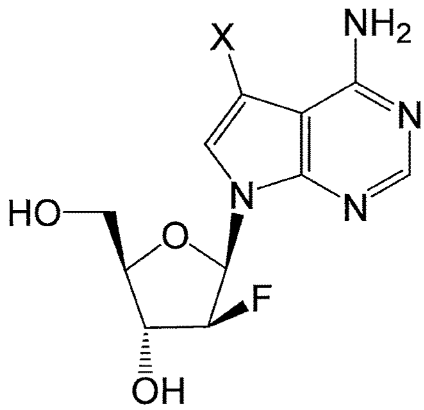

(2)

wherein
X represents a chlorine atom, a bromine atom or an iodine atom,
or a pharmaceutically acceptable salt thereof.

[22] An antitumor agent comprising the compound or a pharmaceutically acceptable salt thereof according to [21], as an active ingredient.

[23] A pharmaceutical composition comprising the compound or a pharmaceutically acceptable salt thereof according to [21] and a pharmaceutically acceptable carrier.

[24] The antitumor agent according to [22] or the pharmaceutical composition according to [23], formulated as an oral preparation or an injection.

[25] A method for preventing and/or treating a tumor, comprising administering the compound or a pharmaceutically acceptable salt thereof according to [21] .

[26] The compound or a pharmaceutically acceptable salt thereof according to [21], for preventing and/or treating a tumor.

[27] Use of the compound or a pharmaceutically acceptable salt thereof according to [21], for producing a pharmaceutical composition or an antitumor agent.

[28] The antitumor agent according to [22], the method for preventing and/or treating a tumor according to [25], the compound or a pharmaceutically acceptable salt thereof according to [26] or the use according to [27], wherein the tumor is selected from the group consisting of head and neck cancer (e.g., oral cancer, pharyngeal cancer, laryngeal cancer, nasal cancer, sinus cancer, salivary gland cancer, thyroid cancer), gastrointestinal cancer (e.g., esophageal cancer, stomach cancer, duodenal cancer, liver cancer, biliary tract cancer (e.g., gall bladder/bile duct cancer), pancreatic cancer, colorectal cancer (e.g., colon cancer, rectal cancer)), lung cancer (e.g., non-small cell lung cancer, small cell lung cancer, mesothelioma), breast cancer, genital cancer (e.g., ovarian cancer, uterine cancer (e.g., cervical cancer, endometrial cancer)), urinary cancer (e.g., kidney cancer, bladder cancer, prostate cancer, a testicular tumor), a hematopoietic organ tumor (e.g., leukemia, malignant lymphoma, multiple myeloma), a bone/soft tissue tumor, skin cancer and a brain tumor.

[29] The antitumor agent, method for preventing and/or treating a tumor, compound or a pharmaceutically acceptable salt thereof or the use according to [28], wherein the tumor is a brain tumor.

[30] The antitumor agent or pharmaceutical composition consisting of the compound or a pharmaceutically acceptable salt thereof according to any one of [1] to [7] and [21], which is used in combination with an alkylating agent.

[31] An enhancer of an antitumor effect comprising the compound or a pharmaceutically acceptable salt thereof according to any one of [1] to [7] and [21], for enhancing an antitumor effect of an alkylating agent.

[32] An antitumor agent comprising the compound or a pharmaceutically acceptable salt thereof according to any one of [1] to [7] and [21], for treating a cancer patient having received administration of an alkylating agent.

[33] A method for treating a tumor, comprising administering the compound or a pharmaceutically acceptable salt thereof according to any one of [1] to [7] and [21], which is used in combination with an alkylating agent.

[34] A method for enhancing an antitumor effect of an alkylating agent, comprising administering the compound or a pharmaceutically acceptable salt thereof according to any one of [1] to [7] and [21] to a patient.

[35] A method for preventing and/or treating a tumor, comprising administering an antitumor agent consisting of the compound or a pharmaceutically acceptable salt thereof according to any one of [1] to [7] and [21], and an alkylating agent.

[36] Use of the compound or a pharmaceutically acceptable salt thereof according to any one of [1] to [7] and [21], for treating a tumor, which is administered in combination with an alkylating agent.

[37] Use of the compound or a pharmaceutically acceptable salt thereof according to any one of [1] to [7] and [21],

for enhancing an antitumor effect of an alkylating agent, which is administered in combination with an alkylating agent.

[38] Use of the compound or a pharmaceutically acceptable salt thereof according to any one of [1] to [7] and [21], for treating a tumor, characterized by treating a cancer patient having received administration of an alkylating agent.

[39] Use of the compound or a pharmaceutically acceptable salt thereof according to any one of [1] to [7] and [21], for enhancing an antitumor effect of an alkylating agent, characterized by treating a cancer patient having received administration of an alkylating agent.

[40] Use of the compound or a pharmaceutically acceptable salt thereof according to any one of [1] to [7] and [21], for producing an antitumor agent, wherein the antitumor agent is administered in combination with an alkylating agent.

[41] Use of the compound or a pharmaceutically acceptable salt thereof according to any one of [1] to [7] and [21], for producing an enhancer of an antitumor effect of an alkylating agent, wherein the enhancer of an antitumor effect is administered in combination with the alkylating agent.

[42] Use of the compound or a pharmaceutically acceptable salt thereof according to any one of [1] to [7] and [21], for producing an antitumor agent, wherein the antitumor agent is administered to a cancer patient having received administration of an alkylating agent or a cancer patient to receive administration of an alkylating agent.

[43] Use of the compound or a pharmaceutically acceptable salt thereof according to any one of [1] to [7] and [21], for producing an enhancer of an antitumor effect of an alkylating agent, wherein the enhancer of an antitumor effect is administered to a cancer patient having received administration of an alkylating agent or a cancer patient to receive administration of an alkylating agent.

[44] The antitumor agent according to [30] or [32], the enhancer of an antitumor effect according to [31], the method according to any one of [33] to [35] or the use according to any one of [36] to [43], wherein the alkylating agent is temozolomide.

[45] The antitumor agent according to [30] or [32], the enhancer of an antitumor effect according to [31], the method according to any one of [33] to [35], or the use according to any one of [36] to [43], which is used in combination with a radiation therapy in addition to the alkylating agent.

[46] An antitumor agent consisting of the compound or a pharmaceutically acceptable salt thereof according to any one of [1] to [7] and [21], which is used in combination with a radiation therapy.

[47] An enhancer of an antitumor effect comprising the compound or a pharmaceutically acceptable salt thereof according to any one of [1] to [7] and [21], for enhancing an antitumor effect of a radiation therapy.

[48] An antitumor agent or pharmaceutical composition comprising the compound or a pharmaceutically acceptable salt thereof according to any one of [1] to [7] and [21], for treating a cancer patient having received a radiation therapy.

[49] A method for preventing and/or treating a tumor, comprising the compound or a pharmaceutically acceptable salt thereof according to any one of [1] to [7] and [21], which is used in combination with a radiation therapy.

[50] A method for enhancing an antitumor effect of a radiation therapy, comprising administering the compound or a pharmaceutically acceptable salt thereof according to any one of [1] to [7] and [21] to a patient.

[51] A method for preventing and/or treating a tumor, comprising administering an antitumor agent consisting of the compound or a pharmaceutically acceptable salt thereof according to any one of [1] to [7] and [21] to a cancer patient having received a radiation therapy.

[52] Use of the compound or a pharmaceutically acceptable salt thereof according to any one of [1] to [7] and [21], for treating a tumor, which is used in combination with a radiation therapy.

[53] Use of the compound or a pharmaceutically acceptable salt thereof according to any one of [1] to [7] and [21], for enhancing an antitumor effect, which is used in combination with a radiation therapy.

[54] Use of the compound or a pharmaceutically acceptable salt thereof according to any one of [1] to [7] and [21], for treating a tumor, which treats a cancer patient having received a radiation therapy.

[55] Use of the compound or a pharmaceutically acceptable salt thereof according to any one of [1] to [7] and [21], for enhancing an antitumor effect, which treats a cancer patient having received a radiation therapy.

[56] Use of the compound or a pharmaceutically acceptable salt thereof according to any one of [1] to [7] and [21], for producing an antitumor agent, wherein the antitumor agent is used in combination with a radiation therapy.

[57] Use of the compound or a pharmaceutically acceptable salt thereof according to any one of [1] to [7] and [21], for producing an enhancer of an antitumor effect, wherein the enhancer of an antitumor effect is used in combination with a radiation therapy.

[58] Use of the compound or a pharmaceutically acceptable salt thereof according to any one of [1] to [7] and [21], for producing an antitumor agent, wherein the antitumor agent is administered to a cancer patient having received a radiation therapy.

[59] Use of the compound or a pharmaceutically acceptable salt thereof according to any one of [1] to [7] and [21], for producing an enhancer of an antitumor effect, wherein the enhancer of an antitumor effect is administered to a cancer patient having received a radiation therapy.

[60] The antitumor agent according to [46] or [48], the enhancer of an antitumor effect according to [47], the method according to any one of [49] to [51] or the use according to any one of [52] to [59], which is used in combination with

an alkylating agent in addition to a radiation therapy.
[61] The antitumor agent, enhancer of an antitumor effect, method or use according to [60], wherein the alkylating agent is temozolomide.
[62] A combination comprising: a pharmaceutical composition comprising the compound or a pharmaceutically acceptable salt thereof according to any one of [1] to [7] and [21]; and an alkylating agent.
[63] A combination drug comprising the compound or a pharmaceutically acceptable salt thereof according to any one of [1] to [7] and [21] and an alkylating agent.
[64] The combination according to [62] or the combination drug according to [63], which is used in combination with a radiation therapy.
[65] The combination according to [62] or the combination drug according to [63], formulated as an oral preparation or an injection.

Advantageous Effects of Invention

The novel phosphate ester compound having a pyrrolopyrimidine skeleton or a pharmaceutically acceptable salt thereof is useful as an antitumor agent having excellent safety and exerting a high antitumor effect. Also, the novel phosphate ester compound of the present invention or a pharmaceutically acceptable salt thereof, if it is used in combination with an alkylating agent and/or a radiation therapy, shows an excellent combinational effect.

Brief Description of Drawings

[Figure 1] Figure 1 shows IR (vertical axis) on the final day of evaluation when the Comparative Example 2-compound and Example 2-compound were administered to BALB/cA Jcl-nu/nu mice transplanted with a human brain tumor cell line (U-87MG).
[Figure 2] Figure 2 shows BWC (vertical axis) on individual evaluation days (horizontal axis) when the Comparative Example 2-compound and Example 2-compound were administered to BALB/cA Jcl-nu/nu mice transplanted with a human brain tumor cell line (U-87MG). ○ represents a control group of mice (not treated); ■ represents a group receiving the Comparative Example 2-compound; and ▲ represents a group receiving the Example 2-compound.
[Figure 3] Figure 3 shows RTV (vertical axis) on individual evaluation days (horizontal axis) when the Comparative Example 2-compound was administered to BALB/cA Jcl-nu/nu mice transplanted with a human hematopoietic organ tumor cell line (MV-4-11). ○ represents a control group (not treated); and ■ represents a group receiving the Comparative Example 2-compound.
[Figure 4] Figure 4 shows BWC (vertical axis) on individual evaluation days (horizontal axis) when the Comparative Example 2-compound was administered to BALB/cA Jcl-nu/nu mice transplanted with a human hematopoietic organ tumor cell line (MV-4-11). ○ represents a control group (not treated); and ■ represents a group receiving the Comparative Example 2-compound.
[Figure 5] Figure 5 shows RTV (vertical axis) on individual evaluation days (horizontal axis) when the Example 2-compound was administered to BALB/cA Jcl-nu/nu mice transplanted with a human hematopoietic organ tumor cell line (MV-4-11). ○ represents a control group of mice (not treated); and ▲ represents a group receiving the Example 2-compound.
[Figure 6] Figure 6 shows BWC (vertical axis) on individual evaluation days (horizontal axis) when the Example 2-compound was administered to BALB/cA Jcl-nu/nu mice transplanted with a human hematopoietic organ tumor cell line (MV-4-11). ○ represents a control group (not treated); and ▲ represents a group receiving the Example 2-compound.
[Figure 7] Figure 7 shows changes of lymphocytes (LYMPH) and monocytes (MONO) on the final evaluation day of a group of BALB/cA Jcl-nu/nu mice transplanted with a human hematopoietic organ tumor cell line (MV-4-11) receiving the Example 2-compound and the Comparative Example 2-compound, respectively, relative to a control group (not treated).
[Figure 8] Figure 8 shows cell viability of a human brain tumor cell line (U-87MG) treated with the Comparative Example 2-compound and a radiation therapy singly or in combination.
[Figure 9] Figure 9 shows cell viability of a human brain tumor cell line (U-87MG) treated with the Comparative Example 2-compound and temozolomide (TMZ) singly or in combination.

Description of Embodiments

Now, the present invention will be more specifically described. It should not be construed that the scope of the

present invention is limited to the embodiments described below.

<Definitions of terms>

The terms used in the specification have meanings commonly used in the technical field, unless otherwise specified.

<Compound of formula (1)>

In one embodiment of the present invention, a compound of the present invention represented by the following formula (1):

[Formula 3]

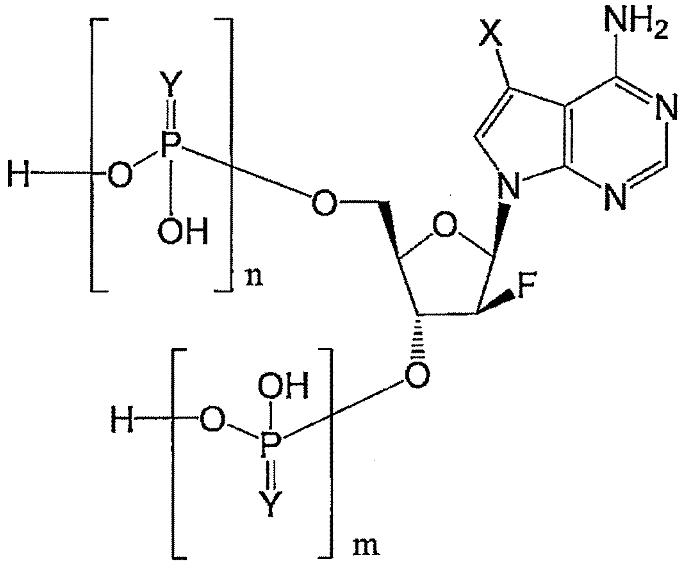

(1)

wherein

X represents a chlorine atom, a bromine atom or an iodine atom,
Ys, which may be the same or different, each represent an oxygen atom or a sulfur atom,
m represents an integer of 0 or 1,
n represents an integer of 0 or 1, and
m + n = 1,

is a novel compound having a pyrrolo[2,3-d]pyrimidine as a basic skeleton.

In one embodiment of the present invention, a compound of the present invention represented by the following formula (1):

[Formula 4]

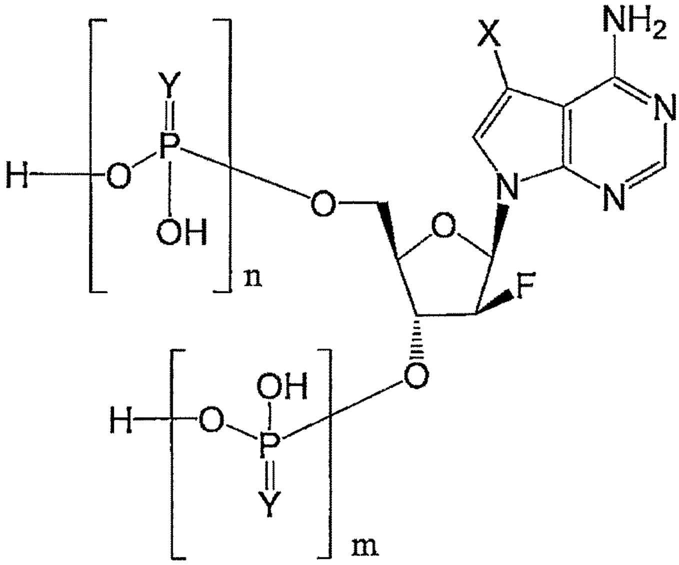

(1)

wherein

X represents a bromine atom or an iodine atom,
Ys, which may be the same or different, each represent an oxygen atom or a sulfur atom,
m represents an integer of 0 or 1,
n represents an integer of 0 or 1, and
m + n = 1,

is a novel compound having a pyrrolo[2,3-d]pyrimidine as a basic skeleton.

In a compound represented by the general formula (1) of the present invention, X represents a chlorine atom, a bromine atom or an iodine atom, preferably, a bromine atom or an iodine atom, and further preferably, an iodine atom.

In a compound represented by the general formula (1) of the present invention, Ys, which may be the same or different, each represent an oxygen atom or a sulfur atom. In a compound of the present invention, Ys preferably each represent an oxygen atom in view of the balance between an antitumor effect and toxicity.

In a compound represented by the general formula (1) of the present invention, n represents an integer of 0 or 1. More specifically, n represents the number of phosphate groups substituted with the hydroxy group at the 5'-position. In a compound of the present invention, n preferably represents 1. A compound of the present invention, if it has a phosphate group as a substituent, can be improved in water solubility and can be used as not only an oral preparation but also an injection.

In one embodiment of the present invention, a compound represented by general formula (1) is preferably a compound where X represents a chlorine atom, a bromine atom or an iodine atom, Y represents an oxygen atom or a sulfur atom, and m + n = 1; and more preferably a compound where X represents a chlorine atom, a bromine atom or an iodine atom, Y represents an oxygen atom, m = 0 and n = 1.

In one embodiment of the present invention, a compound represented by general formula (1) is a compound having the above substituents; preferably, a compound where X represents a bromine atom or an iodine atom; Y represents an oxygen atom, and m + n = 1; and more preferably, a compound X represents a bromine atom or an iodine atom, Y represents an oxygen atom, m = 0 and n = 1.

In one embodiment of the present invention, a compound represented by the general formula (1) of the present invention is preferably a compound where X represents an iodine atom, Y represents an oxygen atom or a sulfur atom, and m + n = 1; and more preferably a compound where X represents an iodine atom, Y represents an oxygen atom, m = 0 and n = 1.

In one embodiment of the present invention, a compound represented by the general formula (1) of the present invention is preferably a compound where X represents a bromine atom, Y represents an oxygen atom or a sulfur atom, and m + n = 1; and more preferably a compound where X represents a bromine atom, Y represents an oxygen atom, m = 0 and n = 1.

Specific examples of the compound of the present invention include, but are not limited to, the following compounds:

(1) ((2R,3R,4S,5R)-5-(4-amino-5-bromo-7H-pyrrolo[2,3-d]pyrimidin-7-yl)-4-fluoro-3-hydroxytetrahydrofuran-2-yl)methyl dihydrogen phosphate,
(2) ((2R,3R,4S,5R)-5-(4-amino-5-iodo-7H-pyrrolo[2,3-d]pyrimidin-7-yl)-4-fluoro-3-hydroxytetrahydrofuran-2-yl)methyl dihydrogen phosphate,
(3) O-(((2R,3R,4S,5R)-5-(4-amino-5-iodo-7H-pyrrolo[2,3-d]pyrimidin-7-yl)-4-fluoro-3-hydroxytetrahydrofuran-2-yl)methyl) O,O-dihydrogen phosphorothioate,
(4) (2R,3R,4S,5R)-5-(4-amino-5-iodo-7H-pyrrolo[2,3-d]pyrimidin-7-yl)-4-fluoro-2-(hydroxymethyl) tetrahydrofuran-3-yl dihydrogen phosphate, and
(5) (2R,3R,4S,5R)-5-(4-amino-5-chloro-7H-pyrrolo[2,3-d]pyrimidin-7-yl)-4-fluoro-3-hydroxytetrahydrofuran-2-yl)methyl dihydrogen phosphate, or pharmaceutically acceptable salts thereof.

A compound of the present invention may be selected from the group consisting of the compounds (1) to (5) or a pharmaceutically acceptable salt thereof and preferably a compound selected from the group consisting of the followings:

(1) ((2R,3R,4S,5R)-5-(4-amino-5-bromo-7H-pyrrolo[2,3-d]pyrimidin-7-yl)-4-fluoro-3-hydroxytetrahydrofuran-2-yl)methyl dihydrogen phosphate; and
(2) ((2R,3R,4S,5R)-5-(4-amino-5-iodo-7H-pyrrolo[2,3-d]pyrimidin-7-yl)-4-fluoro-3-hydroxytetrahydrofuran-2-yl)methyl dihydrogen phosphate, or a pharmaceutically acceptable salt thereof.

In one embodiment of the present invention, there is provided a compound represented by the following formula (2) :

[Formula 5]

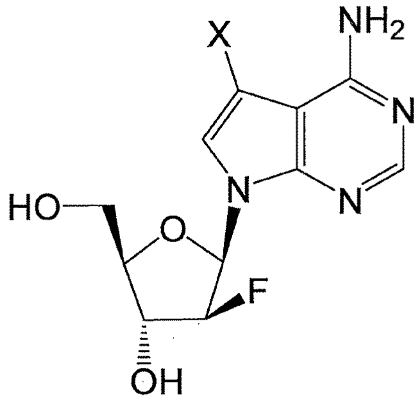

(2)

wherein
X represents a chlorine atom, a bromine atom or an iodine atom,
or a pharmaceutically acceptable salt thereof.

In one embodiment of the present invention, there is provided a compound selected from the group consisting of the followings:

(1) (2R,3R,4S,5R)-5-(4-amino-5-iodo-7H-pyrrolo[2,3-d]pyrimidin-7-yl)-4-fluoro-2-(hydroxymethyl) tetrahydrofuran-3-ol,
(2) (2R,3R,4S,5R)-5-(4-amino-5-bromo-7H-pyrrolo[2,3-d]pyrimidin-7-yl)-4-fluoro-2-(hydroxymethyl) tetrahydrofuran-3-ol, and
(3) (2R,3R,4S,5R)-5-(4-amino-5-chloro-7H-pyrrolo[2,3-d]pyrimidin-7-yl)-4-fluoro-2-(hydroxymethyl) tetrahydrofuran-3-ol,

or a pharmaceutically acceptable salt thereof, as a compound of the present invention.

<Process for producing compound represented by formula (1)>

Now, a process for producing a compound according to the present invention will be described.

A compound represented by formula (1) of the present invention can be produced, for example, by the following production process or a process shown in Examples. However, the process for producing a compound represented by formula (1) of the present invention is not limited to these shown in these reaction examples. The products obtained in individual steps can be subjected to the following steps with or without isolation/purification by an isolation/purification method known in the technical field such as concentration, concentration under reduced pressure, crystallization, solvent extraction, reprecipitation and chromatography. In the following production process, a protecting group may be introduced or removed, if necessarily, regardless of whether a description is made or not, and the order of individual steps may be appropriately changed.

To starting compounds and final products obtained in individual steps, a protecting group that is easily converted into a functional group may be introduced as needed. This is sometimes effective for individual steps or enables to change the order of individual steps. As the protecting group to be used herein, a protecting group described, for example, in literatures ["Protective Groups in Organic Synthesis" written by Greene and Wuts, the fifth edition, John Wiley & Sons Inc., 2014] may be used. The protecting group may be appropriately selected depending on the reaction condition employed in each step. After a reaction is carried out by introducing a protecting group, the protecting group is removed, as needed. In this manner, a desired compound can be obtained.

[Production process 1] Process for producing compound represented by general formula (1a)(in general formula (1), m = 0)

[Formula 6]

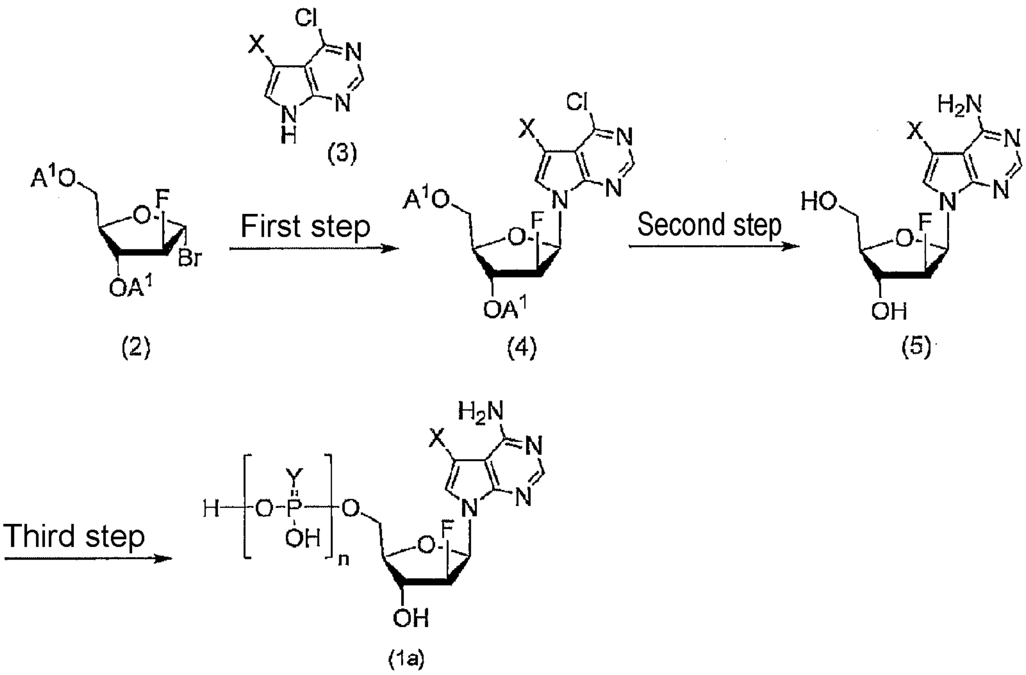


(3) (2) (4) (5) (1a)

wherein X, Y and n are the same as defined above and $A^1$ represents an acyl group

(First step)

In this step, a compound represented by general formula (2) and a compound represented by general formula (3) are reacted in the presence of a base to obtain a compound represented by general formula (4).

The $A^1$ of a compound represented by general formula (2) is not particularly limited as long as it can be deprotected by ammonia. Examples thereof include an acyl group such as a benzoyl group or an acetyl group.

Examples of the base to be used in the reaction include inorganic bases such as sodium hydroxide, sodium hydride, lithium hydroxide, potassium hydride and potassium hydroxide. The solvent to be used in the reaction is not particularly limited as long as it does not affect the reaction. Examples of the solvent include acetonitrile, dioxane,

tetrahydrofuran, N,N-dimethylacetamide, N,N-dimethylformamide and dimethyl sulfoxide. These solvents can be used alone or as a mixture. In the reaction, if necessary, an organic tertiary amine such as tris(2-(2-methoxyethoxy)ethyl)amine may be used. In the reaction, a compound represented by general formula (3) is used in an amount of about 0.5 to 20 moles and preferably about 0.7 to 5 moles; a base is used in an amount of 1.0 to 40 moles, preferably about 1.4 to 10 moles; and an organic tertiary amine is used in an amount of 0.01 to 10 moles and preferably about 0.02 to 5 moles, relative to one mole of a compound represented by general formula (2). The reaction temperature is -30 to 100°C and preferably - 20 to 60°C. The reaction time is 0.1 to 48 hours and preferably 1 to 24 hours.

(Second step)

In this step, a compound represented by general formula (4) and ammonia are reacted to successfully produce a compound represented by general formula (5).

The solvent to be used in the reaction is not particularly limited as long as it does not affect the reaction. Examples of the solvent include dioxane, dimethoxyethane, tetrahydrofuran, N,N-dimethylacetamide, dimethyl sulfoxide and water. These solvents can be used alone or as a mixture. In the reaction, ammonia is used in an amount of about 3 to 1000 moles and preferably about 5 to 500 moles relative to one mole of a compound represented by general formula (4). The reaction temperature is 0 to 200°C and preferably 20 to 150°C. The reaction time is 0.1 to 48 hours and preferably 1 to 24 hours.

(Third step)

In this step, a compound represented by general formula (5) and a phosphorylation reagent are reacted to selectively phosphorylate the 5'-position alone to obtain a compound represented by general formula (1a).

The phosphorylation reagent to be used in the reaction is not particularly limited as long as it can selectively phosphorylate the 5'-position. Examples of the phosphorylation reagent include phosphoryl halides such as phosphorus oxychloride, phosphorus thiochloride and phosphorus oxybromide. In the reaction, if necessary, a base may be used. Examples of the base include organic amines such as imidazole, 1-methylimidazole, triethylamine, triethylamine, tripropylamine, diisopropylethylamine, N-methylmorpholine, pyridine, 4-(N,N-dimethylamino)pyridine, lutidine and collidine; and inorganic bases such as sodium hydrogen carbonate, sodium carbonate and potassium carbonate. The solvent to be used in the reaction is not particularly limited as long as it does not affect the reaction. Examples of the solvent include trimethyl phosphate, triethyl phosphate, tripropyl phosphate, dichloromethane, chloroform, ethyl acetate, tetrahydrofuran, dioxane, diethyl ether, benzene, toluene, N,N-dimethylacetamide and dimethylsulfoxide. These solvents can be used alone or as a mixture. In the reaction, a phosphorylation reagent as mentioned above is used in an amount of 0.5 to 20 moles and preferably about 1 to 10 moles, and a base is used in an amount of 0.5 to 20 moles and preferably about 1 to 10 moles, relative to one mole of a compound represented by general formula (5). The reaction temperature is -30 to 100°C and preferably -20 to 60°C. The reaction time is 0.1 to 100 hours and preferably 1 to 48 hours.

[Production Process 2] Process for producing compound represented by general formula (1b)(in general formula (1), n = 0)

[Formula 7]

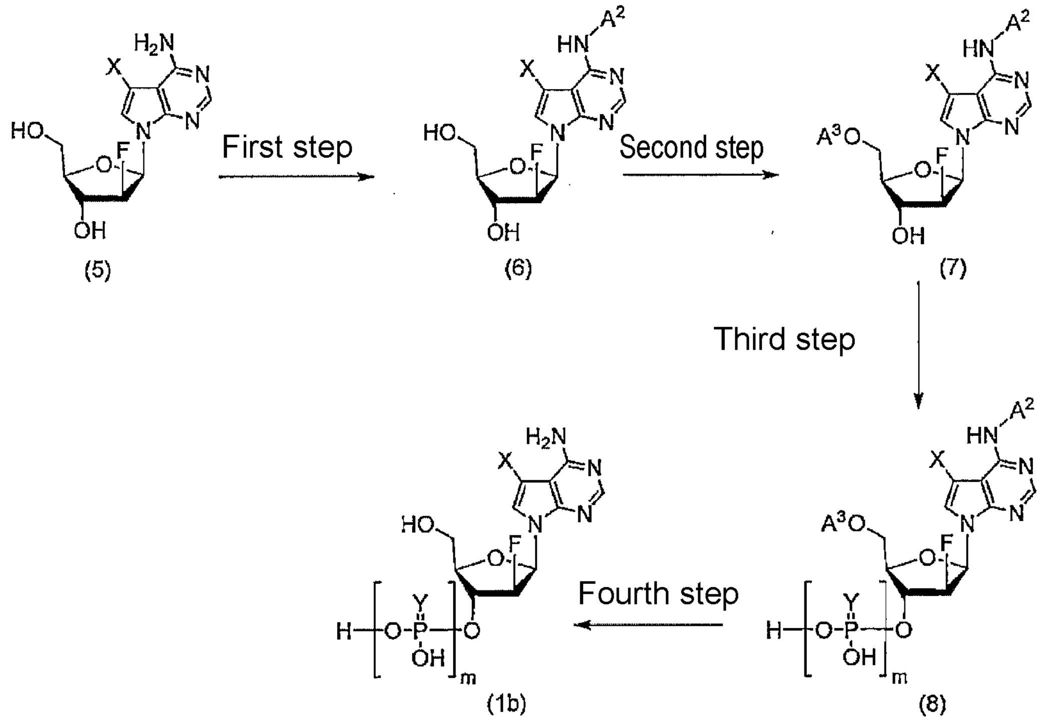


wherein X, Y and m are the same as defined above; and $A^2$ and $A^3$ each represent an acyl group.

(First step)

In this step, a nucleoside compound represented by general formula (5) or a salt thereof and a reagent for introducing a protecting group of an amino group are reacted to selectively protect the amino group alone to obtain a compound represented by general formula (6).

The protecting group selected in this reaction is not particularly limited as long as it can be removed in a basic condition. Examples of the protecting group include acyl halides such as benzoyl chloride, p-chlorobenzoyl chloride and acetyl chloride, represented by $A^2$-Z (Z represents a leaving group such as a halogen atom).

In this reaction, in order to selectively protect an amino group, trimethylsilylation is carried out by using trimethylchlorosilane to previously protect two hydroxy groups in a reaction system; thereafter, $A^2$-Z is reacted with an amino group in the same reaction system; and then, further reacted with ammonia in the same reaction system to remove the trimethylsilyl group. In this manner, a compound represented by general formula (6) is obtained. The solvent to be used in the reaction is not particularly limited as long as it does not affect the reaction. Examples of the solvent include dichloromethane, chloroform, ethyl acetate, tetrahydrofuran, dioxane, diethyl ether, benzene, toluene, N,N-dimethylacetamide, N,N-dimethylformamide and dimethyl sulfoxide. These solvents can be used alone or as a mixture. In the reaction, if necessary, a base may be used. Examples of the base include organic amines such as imidazole, 1-methylimidazole, triethylamine, triethylamine, tripropylamine, diisopropylethylamine, N-methylmorpholine, pyridine, 4-(N,N-dimethylamino)pyridine, lutidine and collidine; and inorganic bases such as sodium hydrogen carbonate, sodium carbonate and potassium carbonate. A base alone may be used as a solvent. In the reaction, trimethylchlorosilane is used in amount of about 1 to 20 moles, and preferably about 1 to 10 moles; $A^2$-Z as mentioned above is used in an amount of about 1 to 20 moles and preferably about 1 to 10 moles; a base is used in an amount of about 1 to 1000 moles and preferably about 1 to 500 moles; and ammonia is used in an amount of about 1 to 10000 moles and preferably about 1 to 1000 moles, relative to one mole of a compound represented by general formula (5). The reaction temperature is -30 to 100°C and preferably -10 to 60°C. The reaction time is 0.1 to 100 hours and preferably 1 to 48 hours.

(Second step)

In this step, a compound represented by general formula (6) is reacted with a reagent for protection to selectively

protect only the hydroxy group at the 5'-position to obtain a compound represented by general formula (7).

The protecting group to be selected in this reaction is not particularly limited as long as it is removed simultaneously with $A^2$ in a basic condition. Examples of a reagent for introducing a protecting group include acyl halides such as benzoyl chloride, p-chlorobenzoyl chloride and acetyl chloride, represented by $A^3$-Z (Z represents a leaving group such as a halogen atom). The solvent to be used in the reaction is not particularly limited as long as it does not affect the reaction. Examples of the solvent include dichloromethane, chloroform, ethyl acetate, tetrahydrofuran, dioxane, diethyl ether, benzene, toluene, N,N-dimethylacetamide, N,N-dimethylformamide and dimethyl sulfoxide. These solvents can be used alone or as a mixture. In the reaction, if necessary, a base may be used. Examples of the base include organic amines such as imidazole, 1-methylimidazole, triethylamine, triethylamine, tripropylamine, diisopropylethylamine, N-methylmorpholine, pyridine, 4-(N,N-dimethylamino)pyridine, lutidine and collidine; and inorganic bases such as sodium hydrogen carbonate, sodium carbonate and potassium carbonate. A base alone may be used as a solvent. In the reaction, $A^3$-Z is used in an amount of about 1 to 20 moles and preferably about 1 to 10 moles and a base is used in an amount of about 1 to 1000 moles and preferably about 1 to 500 moles, relative to one mole of a compound represented by general formula (6). The reaction temperature is -30 to 100°C and preferably -10 to 60°C. The reaction time is 0.1 to 100 hours and preferably 1 to 48 hours.

(Third step)

In this step, a compound represented by general formula (7) is reacted with a phosphorylation reagent to phosphorylate a hydroxy group at the 3'-position to obtain a compound represented by general formula (8).

Examples of the phosphorylation reagent to be used in this reaction include phosphoryl halides such as phosphorus oxychloride, phosphorus thiochloride and phosphorus oxybromide. The solvent to be used in the reaction is not particularly limited as long as it does not affect the reaction. Examples of the solvent include trimethyl phosphate, triethyl phosphate, tripropyl phosphate, dichloromethane, chloroform, ethyl acetate, tetrahydrofuran, dioxane, diethyl ether, benzene, toluene, N,N-dimethylacetamide and dimethylsulfoxide. These solvents can be used alone or as a mixture. In the reaction, a phosphorylation reagent as mentioned above is used in an amount of 0.5 to 20 moles and preferably about 1 to 10 moles relative to one mole of a compound represented by general formula (7). The reaction temperature is -30 to 100°C and preferably -20 to 60°C. The reaction time is 0.1 to 100 hours and preferably 1 to 48 hours.

(Fourth step)

In this step, a reagent for removing a protecting group is reacted with a compound represented by general formula (8). In this manner, protecting groups $A^2$ and $A^3$ are removed to obtain a compound represented by general formula (1b). The solvent to be used is not particularly limited as long as it does not affect the reaction. Examples of the solvent include methanol, ethanol, propanol, isopropanol, tetrahydrofuran, dioxane, diethyl ether, N,N-dimethylacetamide and water. These solvents can be used alone or as a mixture. Examples of the reagent for removing a protecting group to be used include monoalkyl amines such as methyl amine, ethyl amine and propyl amine; and inorganic bases such as sodium hydrogen carbonate, sodium carbonate, potassium carbonate, sodium hydroxide, potassium hydroxide and lithium hydroxide. In the reaction, the reagent for removing a protecting group is used in an amount of 1 to 10000 moles and preferably about 1 to 1000 moles relative to one mole of a compound represented by general formula (8). The reaction temperature is -30 to 100°C and preferably -20 to 60°C. The reaction time is 0.1 to 100 hours and preferably 0.2 to 48 hours.

As described above, a compound represented by general formula (1) or a pharmaceutically acceptable salt thereof, in particular, a compound represented by general formula (1a) having a phosphate at the 5'-position or a compound represented by general formula (1b) having a phosphate at the 3'-position can be produced.

The compound represented by general formula (1) or a pharmaceutically acceptable salt thereof thus produced can be purified by a method commonly used in the technical field. Specific examples of the purification method include, but are not limited to, fractionation by silica gel chromatography or reversed phase chromatography and extraction with an organic layer and a water layer.

The compound of the present invention is a novel phosphate ester compound having a pyrrolopyrimidine skeleton or a pharmaceutically acceptable salt thereof, in this sense, a novel nucleotide derivative. The compound of the present invention can be used as a prodrug, since the compound is converted into an active nucleoside derivative having an antitumor effect by removing a phosphate *in vivo.* Since pharmacokinetics that the active nucleoside derivative originally has can be further improved by the mechanism, and additionally, toxicity that the active nucleoside derivative has can be reduced. Because of this, the compound of the present invention can be used as an extremely excellent antitumor agent.

Reduction of toxicity due to a compound of the present invention is realized in any animal species to which the compound of the present invention is administered. Whether toxicity is reduced or not can be confirmed by checking

suppressions of, e.g., weight loss and reduction of blood components in the animal species receiving the compound.

If a compound of the present invention has isomers such as an optical isomer, a stereoisomer, a rotational isomer and a tautomer, individual isomers and a mixture of isomers are included in the compound of the present invention, unless otherwise specified.

A salt of a compound of the present invention refers to a pharmaceutically acceptable salt such as a base addition salt or an acid addition salt.

A compound of the present invention or a pharmaceutically acceptable salt thereof may be present in amorphous form or crystal form. A compound or a pharmaceutically acceptable salt thereof, even if it has a single crystalline form or a polymorphic mixture, is included in the compound of the present invention or a pharmaceutically acceptable salt thereof. A compound of the present invention or a pharmaceutically acceptable salt thereof may be a solvate (for example, hydrate) or a non-solvate. Both a solvate and a non-solvate thereof are included in the compound of the present invention or a pharmaceutically acceptable salt thereof. A compound labeled with, e.g., isotopes (for example, $^{3}H$, $^{14}C$, $^{35}S$, $^{125}I$) are included in the compound of the present invention or a pharmaceutically acceptable salt thereof.

The compound of the present invention or a pharmaceutically acceptable salt thereof to be used as a medical drug can be produced into various dosage forms depending on prevention or therapeutic purpose by adding, if necessary, a pharmaceutically acceptable carrier. Examples of dosage form of the medical drug include an oral preparation, an injection, a suppository, an ointment and a patch. Preferably an oral preparation or an injection is employed and more preferably an injection is employed. In another embodiment, an oral preparation is more preferably employed as the dosage form of the medical drug.

Examples of the dosage form of the compound of the present invention include an oral preparation or an injection containing the compound according to any one of (1) to (5):

(1) ((2R,3R,4S,5R)-5-(4-amino-5-bromo-7H-pyrrolo[2,3-d]pyrimidin-7-yl)-4-fluoro-3-hydroxytetrahydrofuran-2-yl)methyl dihydrogen phosphate
(2) ((2R,3R,4S,5R)-5-(4-amino-5-iodo-7H-pyrrolo[2,3-d]pyrimidin-7-yl)-4-fluoro-3-hydroxytetrahydrofuran-2-yl)methyl dihydrogen phosphate
(3) O-(((2R,3R,4S,5R)-5-(4-amino-5-iodo-7H-pyrrolo[2,3-d]pyrimidin-7-yl)-4-fluoro-3-hydroxytetrahydrofuran-2-yl)methyl) O,O-dihydrogen phosphorothioate
(4) (2R,3R,4S,5R)-5-(4-amino-5-iodo-7H-pyrrolo[2,3-d]pyrimidin-7-yl)-4-fluoro-2-(hydroxymethyl) tetrahydrofuran-3-yl dihydrogen phosphate
(5) (2R,3R,4S,5R)-5-(4-amino-5-chloro-7H-pyrrolo[2,3-d]pyrimidin-7-yl)-4-fluoro-3-hydroxytetrahydrofuran-2-yl)methyl dihydrogen phosphate or a pharmaceutically acceptable salt thereof.

The various dosage forms of the compound of the present invention are preferably an oral preparation of the compound according to (1) or a pharmaceutically acceptable salt thereof, an injection of the compound according to (1) or a pharmaceutically acceptable salt thereof, an oral preparation of the compound according to (2) or a pharmaceutically acceptable salt thereof, or an injection of the compound according to (2) or a pharmaceutically acceptable salt thereof.

These dosage forms can be each prepared by a method known to those skilled in the art.

The term of "treating" or "treatment" used in the specification includes applying a treatment for the purpose of curing cancer or ameliorating a symptom of cancer, suppressing progression, occurrence or recurrence of cancer, or mitigating a symptom.

The term "effective amount" used in the specification refers to the amount of a pharmaceutically active medical agent sufficient to induce a biological or medical response in a tissue, a system, or in an animal or a human. The effective amount is determined by a researcher, a veterinarian, a doctor or other clinicians. In one embodiment of the present invention, the "effective amount" refers to the amount of an medical-drug active ingredient sufficient to mitigate at least one clinical symptom in a human patient. In one embodiment of the present invention, "effective amount" may be a "prophylactically effective amount", i.e., an amount sufficient to prevent cancer. In one embodiment of the present invention, the "effective amount" of the medical agent which is administered in combination with an alkylating agent can be appropriately reduced from the effective amount of the medical agent singly administered in consideration of not only medicinal effects of both a compound of the present invention or a pharmaceutically acceptable salt thereof and the alkylating agent, but also side effects of them. In one embodiment of the present invention, the "effective amount" of the medical agent which is used in combination with a radiation therapy can be appropriately reduced from the effective amount of the medical agent singly administered in consideration of not only effects of both a compound of the present invention or a pharmaceutically acceptable salt thereof and the radiation therapy, but also side effects of them.

The term "subject" used in the specification includes not only a mammal but also a non-mammal, preferably a human. In one embodiment of the present invention, the subject is a human patient, more specifically, can be a human diagnosed to need a treatment for clinical symptoms or medical conditions associated with cancer disclosed in the specification. The subject may sometimes need an existing treatment for a cancer or a prophylactic treatment for preventing or reducing a risk of developing cancer. The "necessity" of the subject for an existing treatment or a prophylactic treatment for a cancer used in the specification, includes not only necessity determined by a medical professional but also a desire by the patient for the treatment.

In one embodiment of the present invention, there is provided an antitumor agent comprising a compound of the present invention or a pharmaceutically acceptable salt thereof as an active ingredient. In another embodiment of the present invention, there is provided a method for preventing and/or treating a tumor, comprising administering a compound of the present invention or a pharmaceutically acceptable salt thereof to a subject in need thereof. In further another embodiment of the present invention, there is provided use of a compound of the present invention or a pharmaceutically acceptable salt thereof for producing an antitumor agent. In further still another embodiment of the present invention, there is provided a compound of the present invention or a pharmaceutically acceptable salt thereof for use in prevention and/or treatment of a tumor.

In one embodiment of the present invention, there is provided a pharmaceutical composition comprising a compound of the present invention or a pharmaceutically acceptable salt thereof. A pharmaceutical composition according to one embodiment of the present invention comprises a compound of the present invention or a pharmaceutically acceptable salt thereof and a pharmaceutically acceptable carrier. In another embodiment of the present invention, there is provided use of a compound of the present invention or a pharmaceutically acceptable salt thereof for producing a pharmaceutical composition. In another one embodiment of the present invention, there is provided a compound of the present invention or a pharmaceutically acceptable salt thereof for use as a medical drug.

In one embodiment of the present invention, a compound of the present invention or a pharmaceutically acceptable salt thereof can be used in combination with an alkylating agent and/or a radiation therapy.

In one embodiment of the present invention, there is provided a compound of the present invention or a pharmaceutically acceptable salt thereof, which is administered in combination with an alkylating agent. In another embodiment of the present invention, there is provided a compound of the present invention or a pharmaceutically acceptable salt thereof, which is administered in combination with an alkylating agent, for producing an antitumor agent. In another one embodiment of the present invention, there is provided a compound of the present invention or a pharmaceutically acceptable salt thereof, which is administered in combination with an alkylating agent, for treating a tumor.

In the present invention, the "alkylating agent" is active *in vivo* against cancer and an optional medical-drug active ingredient different from a compound of the present invention (or a pharmaceutically acceptable salt thereof). Examples of the alkylating agent to be used in combination include a prodrug of the alkylating agent to be used in combination, a free acid, a free base and a pharmaceutically acceptable salt. Generally, an optional and appropriate additional anti-cancer drug can be used in any combination with a compound of the present invention or a pharmaceutically acceptable salt thereof in a single-dose preparation (for example, a combination of fixed-dosage drugs) or separately in one or more dosage forms. The single-dose preparation enables simultaneous administration of medical-drug active ingredients (simultaneous administration of different medical-drug active ingredients), sequential administrations or separate administrations thereof to a subject. In a specific embodiment, a compound of the present invention and an alkylating agent are administered in combination at intervals of several minutes, several hours or several days. In one embodiment, a single or a plurality of additional anti-cancer drugs are included in a pharmaceutical product, as mentioned above.

In one embodiment of the present invention, there is provided a combination product, which contains a pharmaceutical composition comprising a compound of the present invention or a pharmaceutically acceptable salt thereof and an alkylating agent. The pharmaceutical composition comprising a compound of the present invention or a pharmaceutically acceptable salt thereof, and the alkylating agent, which are contained in the combination product, can be simultaneously (simultaneous administration of different medical-drug active ingredients), sequentially or separately administered at intervals of several minutes, several hours, several days, several weeks or several months.

In the present invention, examples of the "alkylating agent" include, but are not limited to, chlorambucil, chlornaphazine, chlorophosphamide, cytophosphane, estramustine, ifosfamide, mannomustine, mechlorethamine, mechlorethamine hydrochloride, melphalan, novembichin, phenesterine, prednimustine, trimustine chloroethylamine, trofosfamide, and uracil mustard; alkyl sulfonates such as busulfan, improsulfan and piposulfan; nitrosoureas such as carmustine, chlorozotocin, fotemustine, lomustine, nimustine, ranimustine, streptozotocin and TA-07; ethylene imines such as altretamine, thiotepa, triethylenemelamine, triethylenethiophosphoramide, triethylenephosphoramide and trimethylolomelamine; methylmelamine; ambamustine; bendamustine; dacarbazine; etoglucid; irofulven; mafosfamide; mitobronitol; mitolactol; pipobroman; procarbazine; temozolomide; treosulfan; triaziquone and dianhydrogalactitol.

In a preferred embodiment of the present invention, the "alkylating agent" is chlorambucil, estramustine, ifosfamide, melphalan, carmustine, fotemustine, lomustine, nimustine, ranimustine, altretamine, bendamustine, dacarbazine, procarbazine, temozolomide or dianhydrogalactitol. In a further preferred embodiment of the present invention, the "alkylating agent" is estramustine, carmustine, lomustine, nimustine, ranimustine, bendamustine, procarbazine, temozolomide or dianhydrogalactitol. In the most preferred embodiment of the present invention, the "alkylating agent" is temozolomide.

In one embodiment of the present invention, these "alkylating agents" may be used singly or in combination.

In one embodiment of the present invention, an alkylating agent can be administered in combination and a radiation therapy is further used in combination.

In one embodiment of the present invention, there is provided a compound of the present invention or a pharmaceutically acceptable salt thereof, which is used in combination with a radiation therapy. In another embodiment of the present invention, there is provided use of a compound of the present invention or a pharmaceutically acceptable salt thereof, which is used in combination with a radiation therapy, for producing an antitumor agent. In another one embodiment of the present invention, there is provided a compound of the present invention or a pharmaceutically acceptable salt thereof, which is used in combination with a radiation therapy, for treating a tumor.

Techniques for applying a radiation therapy are widely used in the technical field and described, for example, in the "Radiation Therapy Planning Guidelines 2016". These techniques can be used in the invention described in the specification.

Radiation therapies are roughly divided into an external radiation therapy and an internal radiation therapy. The external radiation therapy refers to a therapy for treating cancer by applying radiation from outside of the body, whereas, the internal radiation therapy is a therapy for treating cancer by applying radiation from the interior of the body. In the external radiation therapy, a therapy or irradiation method commonly applied such as a high-energy radiotherapy, a three-dimensional conformal radiotherapy, intensity-modulated radiotherapy (IMRT), image-guided radiotherapy (IGRT), stereotactic radiotherapy (SRT) and stereotactic radiosurgery (SRS), is selected. In the internal radiation therapy, brachytherapy, and a therapy by an unsealed radioisotope are selected. In these radiation therapies, the type of radial ray is selected from an electron ray, X-ray, $\alpha$ (alpha) ray, $\beta$ (beta) ray, $\gamma$ (gamma) ray, proton ray, heavy particle ray, depending on the carcinoma. In the present invention, the radiation therapies and radial rays mentioned above can be used in combination and are not limited to these.

In one embodiment of the present invention, a radiation therapy can be used in combination and an alkylating agent can be administered further in combination.

In one embodiment of the present invention, a compound of the present invention or a pharmaceutically acceptable salt thereof can be used alone for treating cancer, and further used in combination with a radiation therapy. When a compound of the present invention or a pharmaceutically acceptable salt thereof is used alone for treating cancer or when the compound or a salt thereof is used in combination with a radiation therapy, an alkylating agent can be administered in combination.

In one embodiment of the present invention, the compound represented by formula (I) to be used in combination with an alkylating agent and/or a radiation therapy is a compound selected from the group consisting of the following compounds or a pharmaceutically acceptable salt thereof:

(1) ((2R,3R,4S,5R)-5-(4-amino-5-bromo-7H-pyrrolo[2,3-d]pyrimidin-7-yl)-4-fluoro-3-hydroxytetrahydrofuran-2-yl)methyl dihydrogen phosphate
(2) ((2R,3R,4S,5R)-5-(4-amino-5-iodo-7H-pyrrolo[2,3-d]pyrimidin-7-yl)-4-fluoro-3-hydroxytetrahydrofuran-2-yl)methyl dihydrogen phosphate
(3) O-(((2R,3R,4S,5R)-5-(4-amino-5-iodo-7H-pyrrolo[2,3-d]pyrimidin-7-yl)-4-fluoro-3-hydroxytetrahydrofuran-2-yl)methyl) O,O-dihydrogen phosphorothioate
(4) (2R,3R,4S,5R)-5-(4-amino-5-iodo-7H-pyrrolo[2,3-d]pyrimidin-7-yl)-4-fluoro-2-(hydroxymethyl) tetrahydrofuran-3-yl dihydrogen phosphate, and
(5) (2R,3R,4S,5R)-5-(4-amino-5-chloro-7H-pyrrolo[2,3-d]pyrimidin-7-yl)-4-fluoro-3-hydroxytetrahydrofuran-2-yl)methyl dihydrogen phosphate

In one embodiment of the present invention, the compound of the present invention may be more preferably a compound selected from the group consisting of the following compounds or a pharmaceutically acceptable salt thereof:

(1) ((2R,3R,4S,5R)-5-(4-amino-5-bromo-7H-pyrrolo[2,3-d]pyrimidin-7-yl)-4-fluoro-3-hydroxytetrahydrofuran-2-yl)methyl dihydrogen phosphate; and
(2) ((2R,3R,4S,5R)-5-(4-amino-5-iodo-7H-pyrrolo[2,3-d]pyrimidin-7-yl)-4-fluoro-3-hydroxytetrahydrofuran-2-yl)methyl dihydrogen phosphate

In one embodiment of the present invention, the compound represented by formula (2) to be used in combination with an alkylating agent and/or a radiation therapy is a compound selected from the group consisting of the following compounds:

(1) (2R,3R,4S,5R)-5-(4-amino-5-iodo-7H-pyrrolo[2,3-d]pyrimidin-7-yl)-4-fluoro-2-(hydroxymethyl) tetrahydrofuran-3-ol,
(2) (2R,3R,4S,5R)-5-(4-amino-5-bromo-7H-pyrrolo[2,3-d]pyrimidin-7-yl)-4-fluoro-2-(hydroxymethyl) tetrahydrofuran-3-ol, and

(3) (2R,3R,4S,5R)-5-(4-amino-5-chloro-7H-pyrrolo[2,3-d]pyrimidin-7-yl)-4-fluoro-2-(hydroxymethyl) tetrahydrofuran-3-ol,

or a pharmaceutically acceptable salt thereof.

In a specific embodiment of the present invention, a compound of the present invention is administered in an effective amount.

In one embodiment, the alkylating agent is administered in an effective amount.

In a specific embodiment, the alkylating agent to be used in combination with a compound of the present invention or a pharmaceutically acceptable salt thereof is administered in a therapeutically effective amount.

In one embodiment, a compound of the present invention and an alkylating agent are simultaneously administered.

In one embodiment, a compound of the present invention and an alkylating agent are separately administered.

In one embodiment, a compound of the present invention and an alkylating agent are sequentially administered. In one embodiment, a compound of the present invention is administered before administration of an alkylating agent. In one embodiment, a compound of the present invention is administered after an alkylating agent is administered.

In one embodiment, administration of a compound of the present invention and application of a radiation therapy are simultaneously carried out.

In one embodiment, administration of a compound of the present invention and application of a radiation therapy are sequentially carried out. In one embodiment, a compound of the present invention is administered before a radiation therapy is applied. In one embodiment, a compound of the present invention is administered after a radiation therapy is applied.

In one embodiment, a compound disclosed in the present invention or a pharmaceutically acceptable salt thereof and an alkylating agent are administered in the form of a single pharmaceutical product containing these and at least one pharmaceutically acceptable carrier.

Examples of the pharmaceutically acceptable carrier include various organics or inorganic carriers commonly used as components for pharmaceutical products. Examples of the pharmaceutically acceptable carrier to be blended in a solid formulation include an excipient, a binder, a disintegrant, a lubricant, a coating agent and a colorant. Examples of the pharmaceutically acceptable carrier to be blended in a liquid formulation include a solvent, a dissolution aid, a suspending agent, a tonicity agent, a buffer and a soothing agent. If necessary, pharmaceutical additives such as a preservative, an antioxidant, a sweetener and a stabilizer can also be used.

If an oral solid preparation is prepared, a compound of the present invention and an excipient, if necessary, e.g., a binder, a disintegrant, a lubricant, a colorant and a flavoring agent, are added. Thereafter, the mixture can be formed into tablets, coated tablets, granules, powders and capsules in accordance with a customary method.

If an injection is prepared, a compound of the present invention, a pH regulator, a buffer, a stabilizer, a tonicity agent, a local anesthetic and etc. are added. The mixture can be produced into subcutaneous, intramuscular and intravenous injections in accordance with a customary method.

The effective amount of the compound of the present invention to be blended in each of the above unit dosage form varies depending on, e.g., the symptom of the subject to which the compound is to be applied and the dosage form, is commonly 0.05 to 10000 mg for an oral preparation, 0.01 to 5000 mg for an injection, and 1 to 10000 mg for a suppository, per unit dose form as a prodrug. In one embodiment of the present invention, the effective amount of the compound of the present invention to be blended in the unit dosage form is preferably 0.05 to 1000 mg for an oral preparation, 0.01 to 500 mg for an injection, and 1 to 1000 mg for a suppository, per unit dosage form as a prodrug.

The dosage amount of a medical agent having a dosage form as mentioned above per day varies depending on the symptom, body weight, age and sex of a subject and cannot be simply determined. The dosage amount of a compound of the present invention can be usually 0.05 to 50000 mg per adult (body weight: 50 kg) per day and preferably 0.1 to 10000 mg as a prodrug. The dosage amount is preferably 0.05 to 1000 mg for an oral preparation, 0.01 to 500 mg for an injection and 1 to 1000 mg for a suppository, as a prodrug.

Examples of the tumor to which a compound of the present invention is to be applied include, but are not particularly limit to, head and neck cancer (e.g. oral cancer, pharyngeal cancer, laryngeal cancer, nasal cancer, sinus cancer, salivary gland cancer, thyroid cancer), gastrointestinal cancer (e.g., esophageal cancer, stomach cancer, duodenal cancer, liver cancer, biliary tract cancer (e.g., gall bladder/bile duct cancer), pancreatic cancer, colorectal cancer (e.g., colon cancer, rectal cancer)), lung cancer (e.g., non-small cell lung cancer, small cell lung cancer, mesothelioma), breast cancer, genital cancer (e.g., ovarian cancer, uterine cancer (e.g., cervical cancer, endometrial cancer)), urinary cancer (e.g., kidney cancer, bladder cancer, prostate cancer, a testicular tumor), a hematopoietic organ tumor (e.g., leukemia, malignant lymphoma, multiple myeloma), a bone/soft tissue tumor, skin cancer and a brain tumor.

Examples of the brain tumor to be treated by a compound of the present invention include a metastatic brain tumor and a primary brain tumor.

Examples of the brain tumor include, but are not particularly limited to, metastatic brain tumors (for example, brain metastasis of, e.g., lung cancer, breast cancer, stomach cancer, colorectal cancer, bladder cancer, biliary tract cancer and uterine cancer), pilocytic astrocytoma, diffuse astrocytoma, oligodendroglioma/oligoastrocytoma, anaplastic astrocytoma/anaplastic oligodendroglioma, anaplastic oligoastrocytoma, glioblastoma, ependymoma, anaplastic ependymoma, ganglioglioma, central neurocytoma, medulloblastoma, germinoma, central nervous system malignant lymphoma, meningioma, schwannoma, GH producing pituitary adenoma, PRL producing pituitary adenoma, ACTH producing pituitary adenoma, non-functional pituitary adenoma, craniopharyngioma, chordoma, hemangioblastoma and epidermoid tumor.

Since a compound of the present invention is used as a prodrug, its active compound, i.e., a compound represented by general formula (1) wherein m and n each represents 0, other substituents are defined as the same as above, or a pharmaceutically acceptable salt thereof, is applied to the same tumors as mentioned above.

Since a compound of the present invention is used as a prodrug, the compounds represented by the following formulas, respectively which are the active compounds represented by:

(2R,3R,4S,5R)-5-(4-amino-5-iodo-7H-pyrrolo[2,3-d]pyrimidin-7-yl)-4-fluoro-2-(hydroxymethyl) tetrahydrofuran-3-ol;

(2R,3R,4S,5R)-5-(4-amino-5-bromo-7H-pyrrolo[2,3-d]pyrimidin-7-yl)-4-fluoro-2-(hydroxymethyl) tetrahydrofuran-3-ol; and

(2R,3R,4S,5R)-5-(4-amino-5-chloro-7H-pyrrolo[2,3-d]pyrimidin-7-yl)-4-fluoro-2-(hydroxymethyl) tetrahydrofuran-3-ol

or pharmaceutically acceptable salts thereof, are applied to the same carcinomas as mentioned above.

[Formula 8]

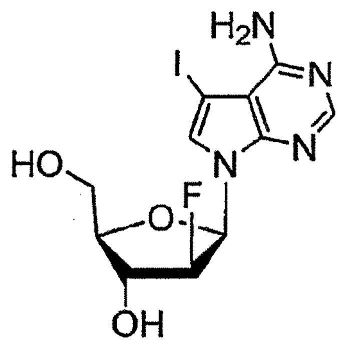

[Formula 9]

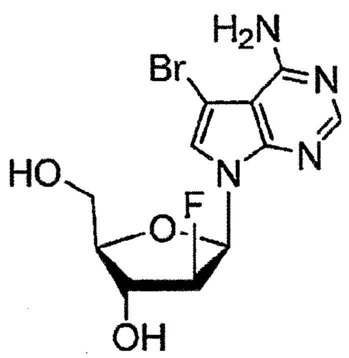

[Formula 10]

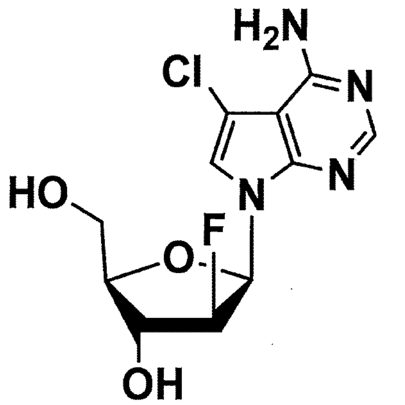

Examples

The present invention will be more specifically described by way of Examples and Test Examples shown below, but the present invention is not limited to these Examples.

As the reagents used in Examples, commercially available reagents were used unless otherwise specified. Silica gel column chromatography and basic silica gel column chromatography were carried out by using prepacked columns manufactured by Shoko Science Co., Ltd. or Biotage Ltd.

Reverse phase preparative HPLC column chromatography was carried out in the following conditions. Injection amount and gradient were appropriately set.

Column: CAPCELL PAK C18 MGIII, 30 × 50 mm, 5 μm, manufactured by OSAKA SODA
UV detection: 254 nm
Column flow rate: 40 mL/minute
Mobile phase: water/acetonitrile (0.1% formic acid)
Injection amount: 0.1-1.0 mL
Gradient water/acetonitrile 10% → 90% (7 minutes)

NMR spectra were measured by use of AL400 (400 MHz; manufactured by JEOL Ltd.(JEOL)), Mercury 400 (400 MHz; manufactured by Agilent Technologies), AVANCE NEO (400 MHz; Bruker), AVANCE III HD (500 MHz; Bruker)-type spectrometer. When tetramethylsilane is contained in a deuterated solvent, tetramethylsilane was used as the internal standard. In the other cases, an NMR solvent was used as the internal standard. All δ values were indicated by ppm.

LC-MS spectra were measured by use of SQD manufactured by Waters in the following two conditions. [M+H]+ values were shown.
MS detection: ESI positive
UV detection: 254 and 210 nm,
Column flow rate: 0.5 mL/minute
Mobile phase: water/acetonitrile (0.1% formic acid)
injection amount: 1 μL
Column: Acquity BEH, 2.1 × 50 mm, 1.7 μm
Gradient:

| Measurement time (minutes) | Water/Acetonitrile (0.1% formic acid) | |
|---|---|---|
| 0 | 95 | 5 |
| 0.1 | 95 | 5 |
| 2.1 | 5 | 95 |
| 3.0 | STOP | |

What are meant by the following abbreviations:

s: singlet
d: doublet
t: triplet
q: quartet
dd: double doublet
m: multiplet
br: broad
brs: broad singlet
DMSO-d6: deuterated dimethyl sulfoxide
D2O: heavy water
HPMC: hydroxypropyl methylcellulose

[Comparative Example 1]

Synthesis of (2R,3R,4S,5R)-5-(4-amino-5-bromo-7H-pyrrolo[2,3-d]pyrimidin-7-yl)-4-fluoro-2-(hydroxymethyl) tetrahydrofuran-3-ol

[Formula 11]

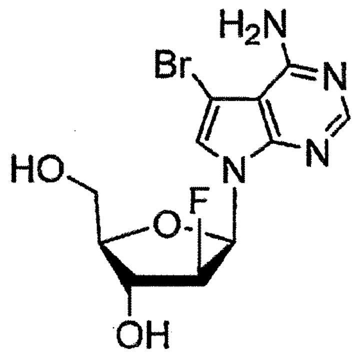

(Step 1)

5-Bromo-4-chloro-7H-pyrrolo[2,3-d]pyrimidine (2.2 g) was suspended in acetonitrile (80 mL). To the mixture, tris(2-(2-methoxyethoxy)ethyl)amine (0.17 mL) and powdery potassium hydroxide (1.1 g) were added. The mixture was stirred while heating at 50°C for 5 hours. To this mixed solution, an acetonitrile (20 mL) solution of ((2R,3R,4S,5R)-3-(benzoyloxy)-5-bromo-4-fluorotetrahydrofuran-2-yl)methyl benzoate (4.5 g) obtained by a method described in the literature (Bioorg. Med. Chem, 20, 2012,5202-5214) was added at room temperature. The resultant mixed solution was stirred at room temperature for 1.5 hours. A part of the solution was taken and subjected to LC-MS analysis. Based on the LC-MS spectrum, the presence of ((2R,3R,4S,5R)-3-(benzoyloxy)-5-(5-bromo-4-chloro-7H-pyrrolo[2,3-d]pyrimidin-7-yl)-4-fluorotetrahydrofuran-2-yl) methyl benzoate was confirmed. Thereafter, purification was carried out in accordance with a method commonly used to obtain the benzoate (5.1 g).

(Step 2)

The benzoate (3.0 g) obtained in step 1 of Comparative Example 1 was suspended in a mixed solution of a 25% aqueous solution of ammonia (16 mL) and 1,4-dioxane (16 mL). The suspension was stirred at 120°C for 5 hours. A part of the solution was taken and subjected to LC-MS analysis. Based on the LC-MS spectrum, the presence of the title compound was confirmed. Thereafter, purification was carried out in accordance with a method commonly used to obtain the title compound (1.3 g).

$^1$H-NMR (DMSO-d6) δ (ppm): 3.55-3.67 (2H, m), 3.74-3.78 (1H, m), 4.29-4.37 (1H, m), 5.02-5.17 (1H, m), 5.06 (1H,

t, J = 5.6 Hz), 5.87 (1H, d, J = 4.8 Hz), 6.52 (1H, dd, J = 4.4, 14 Hz), 6.83 (2H, brs), 7.48 (1H, d, J = 2.0 Hz), 8.09 (1H, s) ESI-MS: m/z 347, 349 (MH+)

[Comparative Example 2]

Synthesis of (2R,3R,4S,5R)-5-(4-amino-5-iodo-7H-pyrrolo[2,3-d]pyrimidin-7-yl)-4-fluoro-2-(hydroxymethyl) tetrahydrofuran-3-ol

[Formula 12]

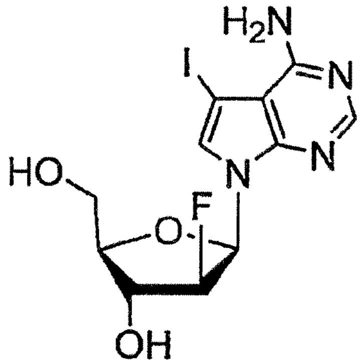

The title compound was synthesized in accordance with a method described in the literature (Bioorg. Med. Chem, 20, 2012, 5202-5214).

[Comparative Example 3]

Synthesis of (2R,3R,4S,5R)-5-(4-amino-5-chloro-7H-pyrrolo[2,3-d]pyrimidin-7-yl)-4-fluoro-2-(hydroxymethyl) tetrahydrofuran-3-ol

[Formula 13]

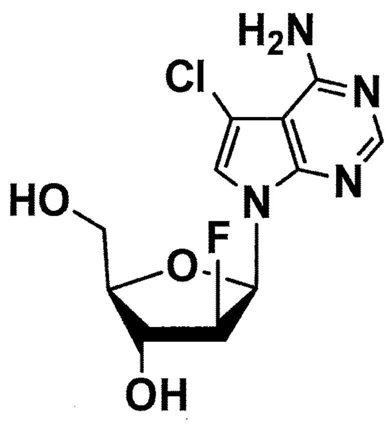

The title compound was synthesized from 5-chloro-4-chloro-7H-pyrrolo[2,3-d]pyrimidine in accordance with the method described in Comparative Example 1. $^1$H-NMR (DMSO-d6) $\delta$ (ppm): 3.63-3.79 (3H, m), 4.34-4.39 (1H, m), 5.09-5.20 (2H, m), 5.90 (1H, s), 6.56 (1H, d, J = 12 Hz), 6.93 (2H, brs), 7.46 (1H, s), 8.12 (1H, s) ESI-MS: m/z 303, 305 (MH+)

[Comparative Example 4]

Synthesis of (2R,3R,4S,5R)-5-(4-amino-5-iodo-7H-pyrrolo[2,3-d]pyrimidin-7-yl)-4,4-difluoro-2-(hydroxymethyl) tetrahydrofuran-3-ol

[Formula 14]

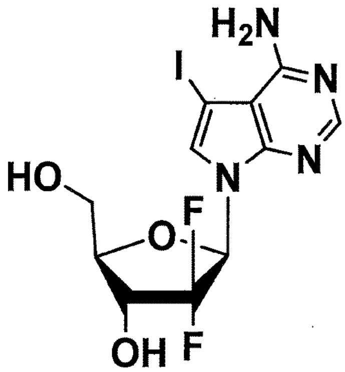

The title compound was synthesized in accordance with a method described in the literature (ChemMedChem, 8, 2013, 832-846).

[Example 1]

Synthesis of ((2R,3R,4S,5R)-5-(4-amino-5-bromo-7H-pyrrolo[2,3-d]pyrimidin-7-yl)-4-fluoro-3-hydroxytetrahydrofuran-2-yl)methyl dihydrogen phosphate

(2R,3R,4S,5R)-5-(4-Amino-5-bromo-7H-pyrrolo[2,3-d]pyrimidin-7-yl)-4-fluoro-2-(hydroxymethyl) tetrahydrofuran-3-ol (300 mg) obtained in Comparative Example 1 was dissolved in triethyl phosphate (5 mL). To the solution, phosphorous oxychloride (0.16 mL) was added under ice cooling. The resultant solution was stirred at 4°C for 12 hours. A part of the solution was taken and subjected to LC-MS analysis. Based on the LC-MS spectrum, the presence of the title compound was confirmed. Thereafter, purification was carried out in accordance with a method commonly used to obtain the title compound (220 mg).

[Example 2]

Synthesis of ((2R,3R,4S,5R)-5-(4-amino-5-iodo-7H-pyrrolo[2,3-d]pyrimidin-7-yl)-4-fluoro-3-hydroxytetrahydrofuran-2-yl)methyl dihydrogen phosphate

The compound (200 mg) described in Comparative Example 2 was dissolved in triethyl phosphate (2.5 mL). To the solution, phosphorous oxychloride (0.11 mL) was added under ice cooling. The resultant solution was stirred at 4°C for 12 hours. A part of the solution was taken and subjected to LC-MS analysis. Based on the LC-MS spectrum, the presence of the title compound was confirmed. Thereafter, purification was carried out in accordance with a method commonly used to obtain the title compound (158 mg).

[Example 3]

Synthesis of O-(((2R,3R,4S,5R)-5-(4-amino-5-iodo-7H-pyrrolo[2,3-d]pyrimidin-7-yl)-4-fluoro-3-hydroxytetrahydrofuran-2-yl)methyl) O,O-dihydrogen phosphorothioate

The compound (100 mg) described in Comparative Example 2 was dissolved in triethyl phosphate (1 mL). To the solution, 2,6-dimethyl pyridine (0.12 mL) and phosphorus thiochloride (0.077 mL) were added under ice cooling. The resultant solution was stirred for 3 hours under ice cooling. A part of the solution was taken and subjected to LC-MS

analysis. Based on the LC-MS spectrum, the presence of the title compound was confirmed. Thereafter, purification was carried out in accordance with a method commonly used to obtain the title compound (99 mg).

[Example 4]

Synthesis of (2R,3R,4S,5R)-5-(4-amino-5-iodo-7H-pyrrolo[2,3-d]pyrimidin-7-yl)-4-fluoro-2-(hydroxymethyl) tetrahydrofuran-3-yl dihydrogen phosphate

(Step 1)

The compound (200 mg) described in Comparative Example 2 was dissolved in pyridine (2.5 mL). To the solution, chlorotrimethylsilane (0.32 mL) was added and the resultant solution was stirred at room temperature for 2 hours. Thereafter, to the reaction solution, benzoyl chloride (0.025 mL) was added and the resultant solution was stirred at room temperature for 3 hours. Thereafter, the reaction solution was cooled with ice, and water (1 mL) and a 30 % aqueous solution of ammonia (1 mL) were added. The resultant solution was stirred at room temperature. A part of the solution was taken and subjected to LC-MS analysis. Based on the LC-MS spectrum, the presence of N-(7-((2R,3S,4R,5R)-3-fluoro-4-hydroxy-5-(hydroxymethyl) tetrahydrofuran-2-yl)-5-iodo-7H-pyrrolo[2,3-d]pyrimidin-4-yl)benzamide was confirmed. Thereafter, purification was carried out in accordance with a method commonly used to obtain the benzamide (105 mg) .

(Step 2)

The benzamide (105 mg) obtained in step 1 of this Example was dissolved in pyridine (2 mL). To the solution, benzoyl chloride (0.025 mL) was added. The solution was stirred at room temperature for 12 hours. A part of the solution was taken and subjected to LC-MS analysis. Based on the LC-MS spectrum, the presence of ((2R,3R,4S,5R)-5-(4-benzamide-5-iodo-7H-pyrrolo[2,3-d]pyrimidin-7-yl)-4-fluoro-3-hydroxytetrahydrofuran-2-yl)methyl benzoate was confirmed. Thereafter, purification was carried out in accordance with a method commonly used to obtain the benzoate (105 mg).

(Step 3)

The benzoate (105 mg) obtained in step 2 of this Example was dissolved in triethyl phosphate (1.5 mL). To the solution, phosphorus oxychloride (0.065 mL) was added under ice cooling. The solution was stirred at room temperature for 36 hours. A part of the solution was taken and subjected to LC-MS analysis. Based on the LC-MS spectrum, the presence of ((2R,3R,4S,5R)-5-(4-benzamide-5-iodo-7H-pyrrolo[2,3-d]pyrimidin-7-yl)-4-fluoro-3-(phosphonoxy)tetrahydrofuran-2-yl)methyl benzoate was confirmed. Thereafter, purification was carried out in accordance with a method commonly used to obtain the benzoate (100 mg).

(Step 4)

The benzoate (100 mg) obtained in step 3 of this Example was dissolved in a 40% aqueous solution of methyl amine (3 mL). The solution was stirred at room temperature for 2 hours. A part of the solution was taken and subjected to LC-MS analysis. Based on the LC-MS spectrum, the presence of the title compound was confirmed. Thereafter, purification was carried out in accordance with a method commonly used to obtain the title compound (21 mg).

[Example 5]

Synthesis of ((2R,3R,4S,5R)-5-(4-amino-5-chloro-7H-pyrrolo[2,3-d]pyrimidin-7-yl)-4-fluoro-3-hydroxytetrahydrofuran-2-yl)methyl dihydrogen phosphate

The title compound (88 mg) was obtained in the same manner as in Example 1 from (2R,3R,4S,5R)-5-(4-amino-5-chloro-7H-pyrrolo[2,3-d]pyrimidin-7-yl)-4-fluoro-2-(hydroxymethyl) tetrahydrofuran-3-ol (100 mg) obtained in Comparative Example 3.

The chemical structures and physical property values of the compounds of Examples 1 to 5 are shown in the following Table 1.

[Table 1]

| Compound No. | Chemical structure | $^1$H-NMR δ (ppm) | MS |
|---|---|---|---|
| Example 1 | 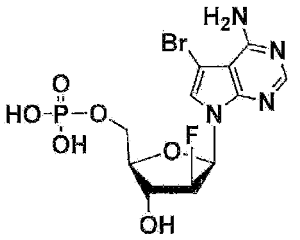 | (DMSO-$d_6$) 3.94-4.09(3H, m), 4.31-4.38(1H, m), 5.03-5.18(1H, m), 6.00(1H, brs), 6.59(1H, dd, J = 4.4, 16 Hz), 6.90(1H, brs), 7.45(1H, d, J = 2 Hz), 8.12 (1H, s) | m/z 427, 429 ($MH^+$) |
| Example 2 | 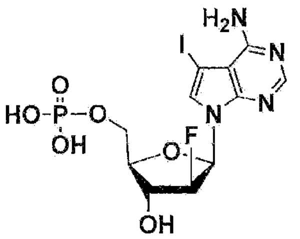 | (DMSO-$d_6$) 3.97-4.12(3H, m), 4.35-4.41(III, m), 5.05-5.20(1H, m), 6.06(1H, brs), 6.59(1H, dd, J = 4.4, 16 Hz), 6.76(1H, brs), 7.48(1H, d, J = 2 Hz), 8.14 (1H, s) | m/z 475 ($MH^+$) |
| Example 3 | 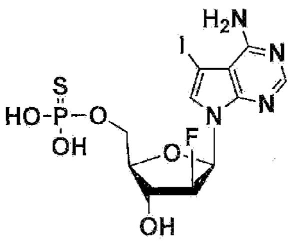 | (DMSO-$d_6$) 3.97-4.18 (3H, m), 4.36-4.43 (1H, m), 5.06-5.21 (1H, m), 6.08 (1H, brs), 6.60 (1H, dd, J = 4.4, 16.3 Hz), 6.77 (2H, brs), 7.51 (1H, d, J = 2.4 Hz), 8.16 (1H, s) | m/z 491 ($MH^+$) |
| Example 4 | 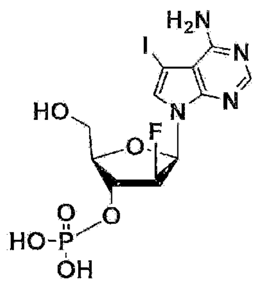 | (DMSO-$d_6$) 358-3.67(2H, m), 3.98-4.05(1H, m), 4.54-4.62(1H, m), 5.12-5.26(1H, m), 6.42(1H, dd, J = 3.2, 20 Hz), 7.47(1H, d, J = 2.4 Hz), 8.09(1H, s) | m/z 475 ($MH^+$) |
| Example 5 | 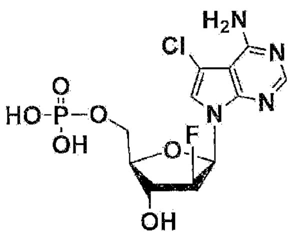 | (DMSO-$d_6$) 3.97-4.12. (3H, m), 4.35-4.41 (1H, m), 5.07-5.22 (1H, m), 6.07 (1H, brs), 6.62 (1H, dd, J = 4.4, 16 Hz), 6.96 (2H, brs), 7.41 (1H, d, J = 1.6 Hz), 8.14 (1H, s) | m/z 383, 385 ($MH^+$) |

The compounds obtained in Examples and Comparative Examples were evaluated by the following tests.

[Test Example 1] Evaluation 1 of presence of prodrug in mouse and absorbency thereof

The dosage amounts of the compound of Comparative Example 1, a triethylamine salt of the compound of Example 1, the compound of Comparative Example 2 and the compound of Example 2 were set to be 50 mg/10 mL/kg, 62 mg/10 mL/kg (50 mg/10 mL/kg in terms of free compound), 3 mg/10 mL/kg, and 3.6 mg/10 mL/kg, respectively. Solutions for administration were prepared with a 0.5% aqueous solution of HPMC so as to satisfy the above dosage amounts. Note that a solution for administration of a triethylamine salt of the compound of Example 1 was prepared by adding 1 molar equivalent of triethylamine.

The dosage amounts of the compound of Comparative Example 3 and the compound of Example 5 were set to be 3 mg/10 mL/kg and 3.8 mg/10 mL/kg, respectively. Solutions thereof for administration were prepared with a 0.5% aqueous solution of HPMC so as to satisfy the above dosage amounts.

Note that, the "mg/10 mL/kg" means as follows: for example "50 mg/10 mL/kg" means that 50 mg of a test compound per body-weight (1 kg) of a mouse was dispersed or dissolved in a solution (10 mL) for administering a compound. The same applies hereinafter.

The solutions for administration prepared above were orally administered to mice (Balb/cA, male) not fasted each by use of an oral administration sonde.

The mice were anesthetized with isoflurane 0.5, 1, 2, 4 and 6 hours after administration and blood was taken from the facial vein by use of a specific needle (animal lancet) and a heparin-coated hematocrit tube.

The blood sampled was immediately centrifuged (13000 rpm, 2 minutes, 4°C) to prepare the plasma samples. After proteins were removed from the plasma samples, the concentrations of the test compound in the plasma samples were measured by LC/MS/MS.

The value of "area under the curve" (AUC) of drug concentration in blood versus time was calculated using analysis software such as Phoenix (registered trademark) WinNonlin (registered trademark).

The AUC (AUC 0-6 hr) values obtained in the time period of 0 to 6 hours after administration are shown in the following tables. More specifically, the results of the compounds of Comparative Examples 1 and 2 and triethylamine salt of Example 1, and the compound of Example 2 are shown in Table 2; whereas, the results of the compounds of Comparative Example 3 and Example 5 are shown in Table 3. In Table 2 and Table 3, "Comparative Example 1", "Comparative Example 2", and "Comparative Example 3" represent the "compound (Br form) of Comparative Example 1", the "compound (I form) of Comparative Example 2" and the "compound (Cl form) of Comparative Example 3", respectively.

[Table 2]

| Compound | Dosage | AUC 0-6 hr (μM · hr) |
|---|---|---|
| Comparative Example 1 | 50 mg/kg (0.14 mmol/kg) | 20.25 |
| Triethyl amine salt of Example 1 | 62 mg/kg (0.12 mmol/kg) | 28.37 in terms of active compound of Comparative Example 1 |
| Comparative Example 2 | 3 mg/kg (7.6 μmol/kg) | 0.21 |
| Example 2 | 3.6 mg/kg (7.6 μmol/kg) | 0.34 in terms of active compound of Comparative Example 2 |

[Table 3]

| Compound | Dosage | AUC 0-6 hr (μM · hr) |
|---|---|---|
| Comparative Example 3 | 3 mg/kg (9.9 μmol/kg) | 2.07 |
| Example 5 | 3.8 mg/kg (9.9 μmol/kg) | 2.02 in terms of active compound of Comparative Example 3 |

From the results shown above, it was found that the phosphate ester compound of Example 1 is a prodrug of the compound (Br form) of Comparative Example 1 serving as an active compound. It was also found that the phosphate ester compound of Example 2 is a prodrug of the compound (I form) of Comparative Example 2 serving as an active compound. Further, the AUC values of the phosphate ester compounds of Examples 1 and 2 were likely to be high compared to those of the corresponding active compounds shown in Comparative Examples 1 to 2 orally administered. It was also found that the compound of Example 5 is a prodrug of the compound (Cl form) of Comparative Example 3 serving as an active compound.

[Test Example 2] Evaluation 2 of presence of prodrug in mouse and absorbency thereof

The dosage amounts of a disodium salt of the compound of Example 1, a disodium salt of the compound of Example 2, a disodium salt of the compound of Example 3, a disodium salt of the compound of Example 4 and a disodium

salt of the compound of Example 5 were set to be 26 mg/5 mL/kg, 12 mg/5 mL/kg, 12 mg/5 mL/kg, 12 mg/5 mL/kg, and 11 mg/5 mL/kg, respectively. Solutions for administration were prepared by dissolving the compounds in physiological saline so as to satisfy the above dosage amounts, respectively. Note that solutions for administration of disodium salts of the compounds of Examples 1 to 4 were prepared by adding 2 molar equivalents of sodium hydroxide.

Mice (Balb/cA, male) not fasted were each immobilized on a specific fixed table, sterilized with ethanol for disinfection. Each of the solutions prepared above was administered through the caudal vein by use of a syringe and a needle.

Blood was taken from the facial vein, 0.25, 0.5, 1, 2, 4 and 6 hours after administration by use of a specific needle (animal lancet) and a heparin-coated hematocrit tube.

The blood sampled were immediately centrifuged (13000 rpm, 2 minutes, 4°C) to prepare the plasma samples. After proteins were removed from the plasma samples, the concentrations of the test compound in the plasma samples were measured by LC/MS/MS.

The value of AUC was calculated using analysis software such as Phoenix (registered trademark), WinNonlin (registered trademark).

The AUC (AUC 0-6 hr) values obtained in the time period of 0 to 6 hours after administration of sodium salts of the compounds of Example 1 to 4 are shown in Table 4; and the AUC (AUC 0-6 hr) values obtained in the time period of 0 to 6 hours after administration of a sodium salt of the compound of Example 5 are shown in Table 5. In Table 4 and Table 5, "Comparative Example 1", "Comparative Example 2" and "Comparative Example 3" represent the "compound (Br form) of Comparative Example 1", the "compound (I form) of Comparative Example 2", and the "compound (Cl form) of Comparative Example 3", respectively.

[Table 4]

| Compound | Dosage | AUC 0-6 hr (μM · hr) |
|---|---|---|
| Disodium salt of Example 1 | 26 mg/kg | 15.9 in terms of active compound of Comparative Example 1 0.15 in terms of Example 1 |
| Disodium salt of Example 2 | 12 mg/kg | 6.07 in terms of active compound of Comparative Example 2 0.64 in terms of Example 2 |
| Disodium salt of Example 3 | 12 mg/kg | 4.28 in terms of active compound of Comparative Example 2 0.18 in terms of Example 3 |
| Disodium salt of Example 4 | 12 mg/kg | 9.76 in terms of active compound of Comparative Example 2 9.35 in terms of Example 4 |

[Table 5]

| Compound | Dosage | AUC 0-6 hr (μM · hr) |
|---|---|---|
| Disodium salt of Example 5 | 11 mg/kg | 6.29 in terms of active compound of Comparative Example 3 0.08 in terms of Example 5 |

From the results shown above, it was found that the phosphate ester compound of Example 1 is a prodrug of the compound (Br form) of Comparative Example 1 serving as an active compound. It was also found that the compounds of Examples 2 to 4 are prodrugs of the compound (I form) of Comparative Example 2 serving as an active compound. It was further found that the compound of Example 5 is a prodrug of the compound (Cl form) of Comparative Example serving as an active compound.

[Test Example 3] Evaluation 1 of growth suppression activity in human tumor cell line

Human tumor cell lines different in type were suspended in mediums, seeded in individual wells of a multi-well plate and cultured. On the day after initiation of culture, serially diluted solutions of a compound were added. Culture was carried out for further 3 days. Cells were counted by a CellTiter-Glo (manufactured by Promega KK) in accordance with the protocol recommended by Promega KK. Cell viability was calculated in accordance with the equation shown below and the concentration (IC50 (μM)) of the compound at which 50% of cell growth is inhibited was obtained.

$$\text{Cell viability (\%)} = (T/C) \times 100$$

T: Intensity of light emitted from a well containing a compound
C: Intensity of light emitted from a well containing no compound

The results of the compound (Br form) of Comparative Example 1 are shown in Table 6; whereas, the results of the compound (I form) of Comparative Example 2 are shown in Table 7. In Table 6 and Table 7, "Comparative Example 1" and "Comparative Example 2" represent "compound (Br form) of Comparative Example 1" and "compound (I form) of Comparative Example 2", respectively.

[Table 6]

| Type | Cell line | Comparative Example 1 | Type | Cell line | Comparative Example 1 |
|---|---|---|---|---|---|
| | | IC50 (μM) | | | IC50 (μM) |
| Human bone tumor | A673 | 0.13 | Human lung cancer | H2126 | 0.11 |
| Human breast cancer | HCC1806 | 0.13 | Human lung cancer | H2170 | 0.26 |
| Human breast cancer | MCF7 | 0.08 | Human lung cancer | H226 | 0.13 |
| Human brain tumor | A172 | 0.14 | Human lung cancer | H23 | 0.05 |
| Human brain tumor | LN229 | 0.63 | Human lung cancer | H441 | 0.14 |
| Human head and neck cancer | CA9-22 | 0.08 | Human lung cancer | H460 | 0.08 |
| Human head and neck cancer | DOK | 0.11 | Human lung cancer | H526 | 0.17 |
| Human hematopoietic organ tumor | HL60 | 0.30 | Human lung cancer | H69 | 0.11 |
| Human hematopoietic organ tumor | K562 | 0.11 | Human lung cancer | Mero82 | 0.46 |
| Human hematopoietic organ tumor | MOLT4 | 0.06 | Human lung cancer | Mero83 | 0.35 |
| Human hematopoietic organ tumor | MV-4-11 | 0.03 | Human lung cancer | MESO1 | 0.41 |
| Human hematopoietic organ tumor | RPMI8226 | 0.34 | Human lung cancer | SDM103T2 | 0.58 |
| Human hematopoietic organ tumor | CCRFCEM | 0.05 | Human lung cancer | Mero48a | 0.16 |
| Human hematopoietic organ tumor | BHL-89 | 0.23 | Human lung cancer | SPC111 | 0.36 |
| Human hematopoietic organ tumor | BC3 | 0.02 | Human ovary cancer | A2780 | 0.05 |
| Human hematopoietic organ tumor | BCP1 | 0.11 | Human pancreatic cancer | BXPC3 | 0.27 |
| Human renal cancer | 786-0 | 0.22 | Human pancreatic cancer | CAPAN2 | 0.05 |
| Human colorectal cancer | DLD1 | 0.05 | Human pancreatic cancer | CFPAC1 | 0.11 |
| Human colorectal cancer | HCT116 | 0.12 | Human pancreatic cancer | MIAPACA2 | 0.07 |
| Human colorectal cancer | HT29 | 0.11 | Human prostate cancer | DU145 | 0.09 |

(continued)

| Type | Cell line | Comparative Example 1 | Type | Cell line | Comparative Example 1 |
|---|---|---|---|---|---|
| | | IC50 (μM) | | | IC50 (μM) |
| Human colorectal cancer | SW48 | 0.13 | Human skin cancer | COLO792 | 0.89 |
| Human colorectal cancer | SW620 | 0.23 | Human stomach cancer | HS746T | 0.25 |
| Human lung cancer | DMS273 | 0.40 | Human stomach cancer | MKN45 | 0.21 |
| Human lung cancer | EBC1 | 0.06 | Human stomach cancer | N87 | 0.03 |
| Human lung cancer | H1703 | 0.02 | Human stomach cancer | NUGC3 | 0.07 |
| Human lung cancer | H1975 | 0.07 | Human bladder cancer | HT1197 | 0.13 |
| Human lung cancer | H2081 | 0.09 | Human bladder cancer | HT1376 | 0.06 |

[Table 7]

| Type | Cell line | Comparative Example 2 |
|---|---|---|
| | | IC50 (μM) |
| Human brain tumor | A172 | 0.03 |
| Human brain tumor | LN229 | 0.02 |
| Human hematopoietic organ tumor | MOLT4 | 0.01 |
| Human hematopoietic organ tumor | MV-4-11 | 0.004 |
| Human hematopoietic organ tumor | CCRFCEM | 0.04 |
| Human colorectal cancer | DLD1 | 0.04 |
| Human colorectal cancer | HCT116 | 0.09 |
| Human colorectal cancer | HT29 | 0.13 |
| Human lung cancer | H1703 | 0.04 |
| Human lung cancer | H69 | 0.06 |
| Human pancreatic cancer | CFPAC1 | 0.05 |
| Human pancreatic cancer | MIAPACA2 | 0.04 |
| Human stomach cancer | MKN45 | 0.13 |

From the results shown above, it was found that the compounds of Comparative Example 1 and Comparative Example 2 have antitumor effects on a wide variety of tumors.

[Test Example 4] Evaluation 2 of growth suppression activity to human tumor cell line

Human tumor cell lines different in type were suspended in mediums, seeded in individual wells of a multi-well plate and cultured. On the day after initiation of culture, serially diluted solutions of a compound were added. Culture was carried out for further 3 days. Cells were counted by a CellTiter-Glo (manufactured by Promega KK) in accordance with the protocol recommended by Promega KK. Cell viability was calculated in accordance with the equation shown below and the concentration (IC50 (nM)) of the compound at which 50% of cell growth is inhibited was obtained.

$$\text{Cell viability (\%)} = (T/C) \times 100$$

T: Intensity of light emitted from a well containing a compound
C: Intensity of light emitted from a well containing no compound

The results of the compounds of Comparative Example 1 and Comparative Example 2 are shown in Table 8; whereas, the results of the compound of Comparative Example 3 are shown in Table 9. In Table 8, "Comparative Example 1" and "Comparative Example 2" represent the "compound (Br form) of Comparative Example 1" and the "compound (I form) of Comparative Example 2", respectively. In Table 9, "Comparative Example 3" represent the "compound (Cl form) of Comparative Example 3".

[Table 8]

| Type | Cell line | Comparative Example 1 | Type | Cell line | Comparative Example 2 |
|---|---|---|---|---|---|
| | | IC50 (nM) | | | IC50 (nM) |
| Human lung cancer | A549 | 24 | Human lung cancer | A549 | 14 |
| Human lung cancer | H1650 | 11 | Human lung cancer | H1650 | 8 |
| Human lung cancer | H2122 | 17 | Human lung cancer | H2122 | 9 |
| Human lung cancer | H358 | 69 | Human lung cancer | H358 | 52 |
| Human lung cancer | HCC827 | 228 | Human lung cancer | HCC827 | 176 |
| Human brain tumor | A431 | 32 | Human brain tumor | A431 | 18 |
| Human colorectal cancer | RKO | 31 | Human breast cancer | SK-BR-3 | 23 |
| Human breast cancer | SK-BR-3 | 26 | | | |

[Table 9]

| Type | Cell line | Comparative Example 3 IC50 (nM) |
|---|---|---|
| Human lung cancer | A549 | 54 |
| Human lung cancer | H1650 | 31 |
| Human lung cancer | H2122 | 58 |
| Human lung cancer | H358 | 273 |
| Human lung cancer | HCC827 | 795 |
| Human brain tumor | A431 | 124 |
| Human colorectal cancer | RKO | 135 |
| Human breast cancer | SK-BR-3 | 88 |

From the results shown above, it was found that the compounds of Comparative Examples 1, 2 and 3 have antitumor effects on a wide variety of tumors.

[Test Example 5] Evaluation 3 of growth suppression activity to human tumor cell line

Human tumor cell lines different in type were suspended in mediums, seeded in individual wells of a multi-well plate and cultured. On the day after initiation of culture, serially diluted solutions of a compound were added. Culture was carried out for further 3 days. Cells were counted by a CellTiter-Glo (manufactured by Promega KK) in accordance with the protocol recommended by Promega KK. Cell viability was calculated in accordance with the equation shown below and the concentration (IC50 (nM)) of a compound at which 50% of cell growth is inhibited was obtained.

$$\text{Cell viability (\%)} = (T/C) \times 100$$

T: Intensity of light emitted from a well containing a compound
C: Intensity of light emitted from a well containing no compound

The results are shown in Table 10. In Table 10, "Comparative Example 2" and "Comparative Example 4" represent the "compound (I form) of Comparative Example 2" and the "compound (difluoro form) of Comparative Example 4", respectively.

[Table 10]

| Type | Cell line | Comparative Example 2 IC50 (nM) | Comparative Example 4 IC50 (nM) |
|---|---|---|---|
| Human lung cancer | A427 | 98 | 1136 |
| Human lung cancer | H520 | 29 | 465 |

From the results shown above, it was found that IC50 of Comparative Example 2-compound is more than 10 times lower than that of Comparative Example 4-compound and has an excellent antitumor effect.

[Test Example 6] Evaluation 1 of antitumor effect and toxicity in mouse

A human brain tumor cell line (U-87 MG) was subcutaneously transplanted in the right chest of each of BALB/cA Jcl-nu/nu mice. After tumor transplantation, the major axes (mm) and minor axes (mm) of the tumors were measured and tumor volumes (TV) were calculated. The mice were assigned to individual groups such that the average TVs of the groups became equal. The day of grouping mice was determined Day 0.

Comparative Example 2-compound (I form) was orally administered in a dose of 12 mg/kg/day every day from Day 1, whereas, Example 2-compound was orally administered in a dose of 14.4 mg/kg/day, every day. Thereafter, administration was continued as long as a weight loss was acceptable and then evaluated. Referring to the following literature, administration followed by evaluation/observation was continued until a weight loss became 20% or more.
LABIO 21, No. 30, OCT. 2007, P27

As the indexes of antitumor effect and toxicity, the tumor volumes (TV) and body weights (BW) of individual groups were evaluated, and a tumor-growth inhibition rate (IR) based on relative tumor volume (RTV) to the volume of Day 0 and a body weight change (BWC) were obtained, respectively, in accordance with the following equations. IRs and BWCs of a control group (no treatment), Example 2-compound administration group and Comparative Example 2-compound administration group were compared.

$$\text{TV (mm}^3) = (\text{major axis} \times \text{minor axis}^2)/2$$

$$\text{RTV} = (\text{TV on evaluation date})/(\text{TV on Day 0})$$

$$\text{IR (\%)} = (1 - (\text{RTV of administration group on evaluation date})/(\text{RTV of control group on evaluation date})) \times 100$$

$$BWC\ (\%) = (BW\ on\ evaluation\ date - BW\ on\ Day\ 0)/(BW\ on\ Day\ 0) \times 100$$

IRs of Comparative Example 2-compound administration group and Example 2-compound administration group on the final day of evaluation (Day 6 in the case where Comparative Example 2-compound was used and Day 13 in the case where Example 2-compound was used) are shown in Figure 1 and BWC versus elapsed days is shown in Figure 2.

As a result of the above evaluations, it was observed that administration is continued longer in Example 2-compound administration group compared to Comparative Example 2-compound administration group, in other words, a high antitumor effect was observed while the effect on body weight was mild.

From the observation, it was found that the compound of Example 2 has a high antitumor effect and is excellent in view of safety.

[Test Example 7] Evaluation 2 of antitumor effect and toxicity in mouse

A human hematopoietic organ tumor cell line (MV-4-11) was subcutaneously transplanted in the right chest of each of BALB/cA Jcl-nu/nu mice. After tumor transplantation, the major axes (mm) and minor axes (mm) of the tumors were measured and tumor volumes (TV) were calculated. The mice were assigned to individual groups such that the average TVs of the groups became equal. The day of grouping mice was determined Day 0.

Comparative Example 2-compound (I form) was orally administered in a dose of 12 mg/kg/day every day from Day 1; whereas, Example 2-compound was continuously administered by use of an osmotic pump (an injector-like device) for 14 days in a dose of 20 mg/kg/day. Thereafter, administration was continued as long as a weight loss was acceptable and then evaluated. Referring to the following literature, administration followed by evaluation/observation was continued until a weight loss became 20% or more.

LABIO 21, No. 30, OCT. 2007, P27

The tumor volumes (TV) and body weights (BW) of individual groups were evaluated. As the indexes of antitumor effect and toxicity, the relative tumor volume (RTV) to that of Day 0 and a body weight change (BWC) relative to that of Day 0 were obtained, respectively, in accordance with the following equations and plotted on a chart. RTV and BWC versus elapsed days were compared among a control group (no treatment), Example 2-compound administration group and Comparative Example 2-compound administration group.

$$TV\ (mm^3) = (major\ axis \times minor\ axis^2)/2$$

$$RTV = (TV\ on\ evaluation\ date)/(TV\ on\ Day\ 0)$$

$$BWC\ (\%) = (BW\ on\ evaluation\ date - BW\ on\ Day\ 0)/(BW\ on\ Day\ 0) \times 100$$

Figure 3 and Figure 4 show RTV and BWC versus elapsed days, respectively, when Comparative Example 2-compound was administered. Figure 5 and Figure 6 shows RTV and BWC versus elapsed days, respectively when Example 2-compound was administered.

On the final day (Day 11 in the case where Comparative Example 2-compound was used, Day 15 in the case where Example 2-compound was used) of evaluation, the blood cell components (lymphocytes: LYMPH (Lymphocytes), monocytes: MONO (monocytes)) of individual groups were analyzed as another index for toxicity. The rate (T/C %) of blood cell component each of an Example 2-compound administration group and a Comparative Example 2-compound administration group relative to that of the control group was obtained in accordance with the following equation. The results are shown in Figure 7.

$$T/C\ (\%) = (\text{Number of blood cell component in administration group}/\text{Number of blood cell component in control group}) \times 100$$

As a result of the above evaluations, it was observed that administration can be continued longer in the Example 2-compound administration group than the Comparative Example 2-compound administration group, in other words, a remarkable antitumor effect (tumor regression was confirmed) was observed while an effect on body weight and hematological toxicity were low.

From the above results, it was demonstrated that the compound of Example 2 has a high antitumor effect and is excellent in view of safety.

[Test Example 8] Evaluation of combination use of compound of the invention and radiation

A human brain tumor cell line (U-87 MG) was suspended in a medium, seeded in individual wells of a multiwell plate and cultured. In the case where radiation exposure and addition of a compound are simultaneously carried out, both treatments were carried out on two days after initiation of culture. In the case where addition of a compound preceded radiation exposure, a serially diluted solution of a compound was added on the day after initiation of culture and radiation exposure was carried out on two days after initiation of culture. In the case where radiation exposure preceded addition of a compound, radiation exposure was carried out on the day after initiation of culture and a serially diluted solution of a compound was added on the two days after initiation of culture. Day 5 after seeding, cells were fixed with a 25% aqueous solution of glutaraldehyde and stained with a staining fluid (0.05 w/v% crystal violet in 20% methanol). After an extract (0.1 N $NaH_2PO_4$: 100% ethanol = 1:1) was added and stirred, an absorbance at 540 nm was measured to obtain a cell count. The cell viability was calculated in accordance with the following equation and statistically processed in accordance with a Student's t-test.

$$\text{Cell viability}\ (\%) = (T/C) \times 100$$

T: Absorbance of a well exposed to radiation or containing a compound
C: Absorbance of a well neither exposed to radiation nor containing a compound

The results are shown in Figure 8.

As a result of the above evaluations, it was observed that even if treatments were carried out in any order (the same-day treatment, compound first and radiation first), cell viability significantly decreased in a combination treatment with the Comparative Example 2-compound and radiation exposure compared to the single treatments.

From the above results, it was demonstrated that the Comparative Example 2-compound produces a combinational antitumor effect with radiation exposure.

[Test Example 9] Evaluation of combinational effect of the compound and temozolomide (TMZ)

A human brain tumor cell line (U-87 MG) was suspended in a medium, seeded in individual wells of a multiwell plate and cultured. In the case where two compounds are simultaneously added, both compounds were added on two days after initiation of culture. In the case where addition of the compound of the present invention preceded, a serially diluted solution of the compound was added on the day after initiation of culture and a serially diluted solution of TMZ was added two days after initiation of culture. In the case where addition of TMZ preceded, a serially diluted solution of TMZ was added on the day after initiation of culture and a serially diluted solution of the compound was added on two days after initiation of culture. Day 5 after seeding, cells were fixed with a 25% aqueous solution of glutaraldehyde and stained with a staining fluid (0.05 w/v% crystal violet in 20% methanol). After an extract (0.1 N $NaH_2PO_4$: 100% ethanol = 1:1) was added and stirred, an absorbance at 540 nm was measured to obtain a cell count. The cell viability was calculated in accordance with the following equation and statistically processed in accordance with a Student's t-test.

$$\text{Cell viability}\ (\%) = (T/C) \times 100$$

T: Absorbance of a well containing TMZ or a compound

C: Absorbance of a well containing neither TMZ nor a compound

The results are shown in Figure 9.

As a result of the above evaluations, even if treatments were carried out in any order (the same-day treatment, compound first and TMZ first), a significant decrease in cell viability was observed in a combination treatment with the Comparative Example 2-compound and TMZ compared to the single treatments.

From the above, it was demonstrated that combination use of the Comparative Example 2-compound and TMZ exerts a combinational antitumor effect.

As described in the foregoing, the compound of the present invention is useful as an antitumor agent showing excellent safety and high antitumor effect. The compound of the present invention exerts an excellent combinational effect when it is used in combination with an alkylating agent and/or radiation therapy.

## Claims

1. A compound represented by the following general formula (1):

[Formula 1]

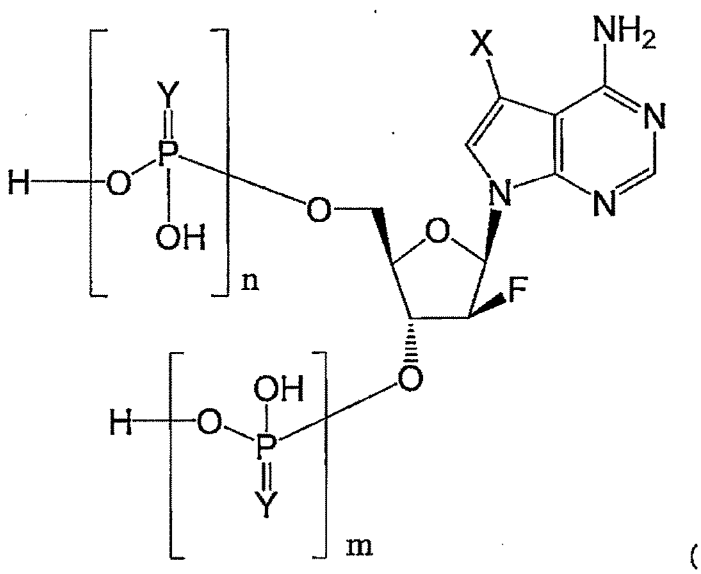

(1)

wherein

X represents a chlorine atom, a bromine atom or an iodine atom,
Ys, which may be the same or different, each represent an oxygen atom or a sulfur atom,
m represents an integer of 0 or 1,
n represents an integer of 0 or 1, and
m + n = 1,

or a pharmaceutically acceptable salt thereof.

2. The compound or a pharmaceutically acceptable salt thereof according to claim 1, wherein X represents a bromine atom or an iodine atom.

3. The compound or a pharmaceutically acceptable salt thereof according to claim 2, wherein X represents an iodine atom.

4. The compound or a pharmaceutically acceptable salt thereof according to claim 3, wherein Y represents an oxygen atom.

5. The compound or a pharmaceutically acceptable salt thereof according to claim 4, wherein m = 0 and n = 1.

6. The compound or a pharmaceutically acceptable salt thereof according to claim 1, wherein X represents a bromine atom or an iodine atom; Y represents an oxygen atom; m = 0; and n = 1.

7. A compound selected from the group consisting of:

(1) ((2R,3R,4S,5R)-5-(4-amino-5-bromo-7H-pyrrolo[2,3-d]pyrimidin-7-yl)-4-fluoro-3-hydroxytetrahydrofuran-2-yl)methyl dihydrogen phosphate;
(2) ((2R,3R,4S,5R)-5-(4-amino-5-iodo-7H-pyrrolo[2,3-d]pyrimidin-7-yl)-4-fluoro-3-hydroxytetrahydrofuran-2-yl)methyl dihydrogen phosphate;
(3) O-(((2R,3R,4S,5R)-5-(4-amino-5-iodo-7H-pyrrolo[2,3-d]pyrimidin-7-yl)-4-fluoro-3-hydroxytetrahydrofuran-2-yl)methyl) O,O-dihydrogen phosphorothioate;
(4) (2R,3R,4S,5R)-5-(4-amino-5-iodo-7H-pyrrolo[2,3-d]pyrimidin-7-yl)-4-fluoro-2-(hydroxymethyl) tetrahydrofuran-3-yl dihydrogen phosphate; and
(5) (2R,3R,4S,5R)-5-(4-amino-5-chloro-7H-pyrrolo[2,3-d]pyrimidin-7-yl)-4-fluoro-3-hydroxytetrahydrofuran-2-yl)methyl dihydrogen phosphate or a pharmaceutically acceptable salt thereof.

8. An antitumor agent comprising the compound or a pharmaceutically acceptable salt thereof according to any one of claims 1 to 7, as an active ingredient.

9. A pharmaceutical composition comprising the compound or a pharmaceutically acceptable salt thereof according to any one of claims 1 to 7 and a pharmaceutical acceptable carrier.

10. The antitumor agent according to claim 8 or the pharmaceutical composition according to claim 9, formulated as an oral preparation or an injection.

11. A method for preventing and/or treating a tumor, comprising administering the compound or a pharmaceutically acceptable salt thereof according to any one of claims 1 to 7.

12. A method for preventing and/or treating a tumor, comprising administering the compound or a pharmaceutically acceptable salt thereof according to any one of claims 1 to 7, formulated as an oral preparation or an injection, to a subject in need thereof.

13. The compound or a pharmaceutically acceptable salt thereof according to any one of claims 1 to 7 for use as a pharmaceutical composition.

14. The compound or a pharmaceutically acceptable salt thereof according to any one of claims 1 to 7 for preventing and/or treating a tumor.

15. The compound or a pharmaceutically acceptable salt thereof according to any one of claims 1 to 7, formulated as an oral preparation or an injection, for use in prevention and/or treatment of a tumor.

16. Use of the compound or a pharmaceutically acceptable salt thereof according to any one of claims 1 to 7, for producing an antitumor agent.

17. Use of the compound or a pharmaceutically acceptable salt thereof according to any one of claims 1 to 7, for producing a pharmaceutical composition.

18. Use of the compound or a pharmaceutically acceptable salt thereof according to any one of claims 1 to 7, for producing an antitumor agent, formulated as an oral preparation or an injection.

19. The antitumor agent according to claim 8, the method for preventing and/or treating a tumor according to claim 11 or 12, the compound or a pharmaceutically acceptable salt thereof according to any one of claims 13 to 15, or the use according to any one of claims 16 to 18, wherein the tumor is selected from the group consisting of head and neck cancer (e.g. oral cancer, pharyngeal cancer, laryngeal cancer, nasal cancer, sinus cancer, salivary gland cancer, thyroid cancer), gastrointestinal cancer (e.g., esophageal cancer, stomach cancer, duodenal cancer, liver cancer, biliary tract cancer (e.g., gall bladder/bile duct cancer), pancreatic cancer, colorectal cancer (e.g., colon

cancer, rectal cancer)), lung cancer (e.g., non-small cell lung cancer, small cell lung cancer, mesothelioma), breast cancer, genital cancer (e.g., ovarian cancer, uterine cancer (e.g., cervical cancer, endometrial cancer)), urinary cancer (e.g., kidney cancer, bladder cancer, prostate cancer, a testicular tumor), a hematopoietic organ tumor (e.g., leukemia, malignant lymphoma, multiple myeloma), a bone/soft tissue tumor, skin cancer and a brain tumor.

**20.** The antitumor agent, method for preventing and/or treating a tumor, compound or a pharmaceutically acceptable salt thereof or use according to claim 19, wherein the tumor is a brain tumor.

**21.** A compound represented by the following general formula (2):

[Formula 2]

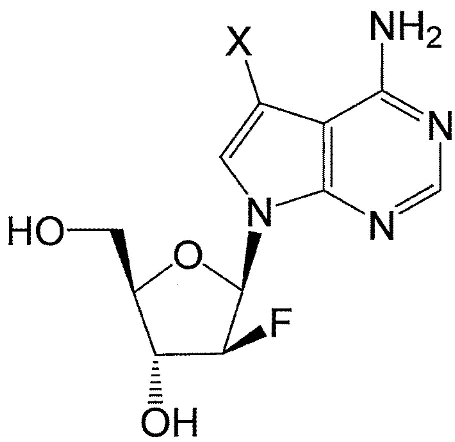

(2)

wherein
X represents a chlorine atom, a bromine atom or an iodine atom,
or a pharmaceutically acceptable salt thereof.

**22.** An antitumor agent comprising the compound or a pharmaceutically acceptable salt thereof according to claim 21, as an active ingredient.

**23.** A pharmaceutical composition comprising the compound or a pharmaceutically acceptable salt thereof according to claim 21 and a pharmaceutically acceptable carrier.

**24.** The antitumor agent according to claim 22 or the pharmaceutical composition according to claim 23, formulated as an oral preparation or an injection.

**25.** A method for preventing and/or treating a tumor, comprising administering the compound or a pharmaceutically acceptable salt thereof according to claim 21.

**26.** The compound or a pharmaceutically acceptable salt thereof according to claim 21, for preventing and/or treating a tumor.

**27.** Use of the compound or a pharmaceutically acceptable salt thereof according to claim 21, for producing a pharmaceutical composition or antitumor agent.

**28.** The antitumor agent according to claim 22, the method for preventing and/or treating a tumor according to claim 25, the compound or a pharmaceutically acceptable salt thereof according to claim 26 or the use according to claim 27, wherein the tumor is selected from the group consisting of head and neck cancer (e.g., oral cancer, pharyngeal cancer, laryngeal cancer, nasal cancer, sinus cancer, salivary gland cancer, thyroid cancer), gastrointestinal cancer (e.g., esophageal cancer, stomach cancer, duodenal cancer, liver cancer, biliary tract cancer (e.g., gall bladder/bile duct cancer), pancreatic cancer, colorectal cancer (e.g., colon cancer, rectal cancer)), lung cancer (e.g., non-small

cell lung cancer, small cell lung cancer, mesothelioma), breast cancer, genital cancer (e.g., ovarian cancer, uterine cancer (e.g., cervical cancer, endometrial cancer)), urinary cancer (e.g., kidney cancer, bladder cancer, prostate cancer, a testicular tumor), a hematopoietic organ tumor (e.g., leukemia, malignant lymphoma, multiple myeloma), a bone/soft tissue tumor, skin cancer and a brain tumor.

**29.** The antitumor agent, method for preventing and/or treating a tumor, compound or a pharmaceutically acceptable salt thereof or use according to claim 28, wherein the tumor is a brain tumor.

**30.** The antitumor agent or pharmaceutical composition consisting of the compound or a pharmaceutically acceptable salt thereof according to any one of claims 1 to 7 and 21, which is used in combination with an alkylating agent.

**31.** An enhancer of an antitumor effect comprising the compound or a pharmaceutically acceptable salt thereof according to any one of claims 1 to 7 and 21, for enhancing an antitumor effect of an alkylating agent.

**32.** An antitumor agent comprising the compound or a pharmaceutically acceptable salt thereof according to any one of claims 1 to 7 and 21, for treating a cancer patient having received administration of an alkylating agent.

**33.** A method for preventing and/or treating a tumor, comprising administering the compound or a pharmaceutically acceptable salt thereof according to any one of claims 1 to 7 and 21, which is used in combination with an alkylating agent.

**34.** A method for enhancing an antitumor effect of an alkylating agent, comprising administering the compound or a pharmaceutically acceptable salt thereof according to any one of claims 1 to 7 and 21 to a patient.

**35.** A method for preventing and/or treating a tumor, comprising administering an antitumor agent consisting of the compound or a pharmaceutically acceptable salt thereof according to any one of claims 1 to 7 and 21, and an alkylating agent.

**36.** Use of the compound or a pharmaceutically acceptable salt thereof according to any one of claims 1 to 7 and 21, for treating a tumor, which is administered in combination with an alkylating agent.

**37.** Use of the compound or a pharmaceutically acceptable salt thereof according to any one of claims 1 to 7 and 21, for enhancing an antitumor effect of an alkylating agent, which is administered in combination with an alkylating agent.

**38.** Use of the compound or a pharmaceutically acceptable salt thereof according to any one of claims 1 to 7 and 21, for treating a tumor, which treats a cancer patient having received administration of an alkylating agent.

**39.** Use of the compound or a pharmaceutically acceptable salt thereof according to any one of claims 1 to 7 and 21, for enhancing an antitumor effect of an alkylating agent, which treats a cancer patient having received administration of an alkylating agent.

**40.** Use of the compound or a pharmaceutically acceptable salt thereof according to any one of claims 1 to 7 and 21, for producing an antitumor agent, wherein the antitumor agent is administered in combination with an alkylating agent.

**41.** Use of the compound or a pharmaceutically acceptable salt thereof according to any one of claims 1 to 7 and 21, for producing an enhancer of an antitumor effect of an alkylating agent, wherein the enhancer of an antitumor effect is administered in combination with an alkylating agent.

**42.** Use of the compound or a pharmaceutically acceptable salt thereof according to any one of claims 1 to 7 and 21, for producing an antitumor agent, wherein the antitumor agent is administered to a cancer patient having received administration of an alkylating agent or a cancer patient to receive administration of an alkylating agent.

**43.** Use of the compound or a pharmaceutically acceptable salt thereof according to any one of claims 1 to 7 and 21, for producing an enhancer of an antitumor effect of an alkylating agent, wherein the enhancer of an antitumor effect is administered to a cancer patient having received administration of an alkylating agent or a cancer patient to receive administration of an alkylating agent.

**44.** The antitumor agent according to claim 30 or 32, the enhancer of an antitumor effect according to claim 31, the

method according to any one of claims 33 to 35, or the use according to any one of claims 36 to 43, wherein the alkylating agent is temozolomide.

**45.** The antitumor agent according to claim 30 or 32, the enhancer of an antitumor effect according to claim 31, the method according to any one of claims 33 to 35, or the use according to any one of claims 36 to 43, which is used in combination with a radiation therapy in addition to the alkylating agent.

**46.** An antitumor agent consisting of the compound or a pharmaceutically acceptable salt thereof according to any one of claims 1 to 7 and 21, which is used in combination with a radiation therapy.

**47.** An enhancer of an antitumor effect comprising the compound or a pharmaceutically acceptable salt thereof according to any one of claims 1 to 7 and 21, for enhancing an antitumor effect of a radiation therapy.

**48.** An antitumor agent or a pharmaceutical composition comprising the compound or a pharmaceutically acceptable salt thereof according to any one of claims 1 to 7 and 21, for treating a cancer patient having received a radiation therapy.

**49.** A method for preventing and/or treating a tumor, comprising the compound or a pharmaceutically acceptable salt thereof according to any one of claims 1 to 7 and 21, which is used in combination with a radiation therapy.

**50.** A method for enhancing an antitumor effect of a radiation therapy, comprising administering the compound or a pharmaceutically acceptable salt thereof according to any one of claims 1 to 7 and 21 to a patient.

**51.** A method for preventing and/or treating a tumor, comprising administrating an antitumor agent consisting of the compound or a pharmaceutically acceptable salt thereof according to any one of claims 1 to 7 and 21 to a cancer patient having received a radiation therapy.

**52.** Use of the compound or a pharmaceutically acceptable salt thereof according to any one of claims 1 to 7 and 21, for treating a tumor, which is used in combination with a radiation therapy.

**53.** Use of the compound or a pharmaceutically acceptable salt thereof according to any one of claims 1 to 7 and 21, for enhancing an antitumor effect, which is used in combination with a radiation therapy.

**54.** Use of the compound or a pharmaceutically acceptable salt thereof according to any one of claims 1 to 7 and 21, for treating a tumor, which treats a cancer patient having received a radiation therapy.

**55.** Use of the compound or a pharmaceutically acceptable salt thereof according to any one of claims 1 to 7 and 21, for enhancing an antitumor effect, which treats a cancer patient having received a radiation therapy.

**56.** Use of the compound or a pharmaceutically acceptable salt thereof according to any one of claims 1 to 7 and 21, for producing an antitumor agent, wherein the antitumor agent is used in combination with a radiation therapy.

**57.** Use of the compound or a pharmaceutically acceptable salt thereof according to any one of claims 1 to 7 and 21, for producing an enhancer of an antitumor effect, wherein the enhancer of an antitumor effect is used in combination with a radiation therapy.

**58.** Use of the compound or a pharmaceutically acceptable salt thereof according to any one of claims 1 to 7 and 21, for producing an antitumor agent, wherein the antitumor agent is administered to a cancer patient having received a radiation therapy.

**59.** Use of the compound or a pharmaceutically acceptable salt thereof according to any one of claims 1 to 7 and 21, for producing an enhancer of an antitumor effect, wherein the enhancer of an antitumor effect is administered to a cancer patient having received a radiation therapy.

**60.** The antitumor agent according to claim 46 or 48, the enhancer of an antitumor effect according to claim 47, the method according to any one of claims 49 to 51 or the use according to any one of claims 52 to 59, which is used in combination with an alkylating agent in addition to the radiation therapy.

**61.** The antitumor agent, enhancer of an antitumor effect, method or use according to claim 60, wherein the alkylating agent is temozolomide.

Fig. 1

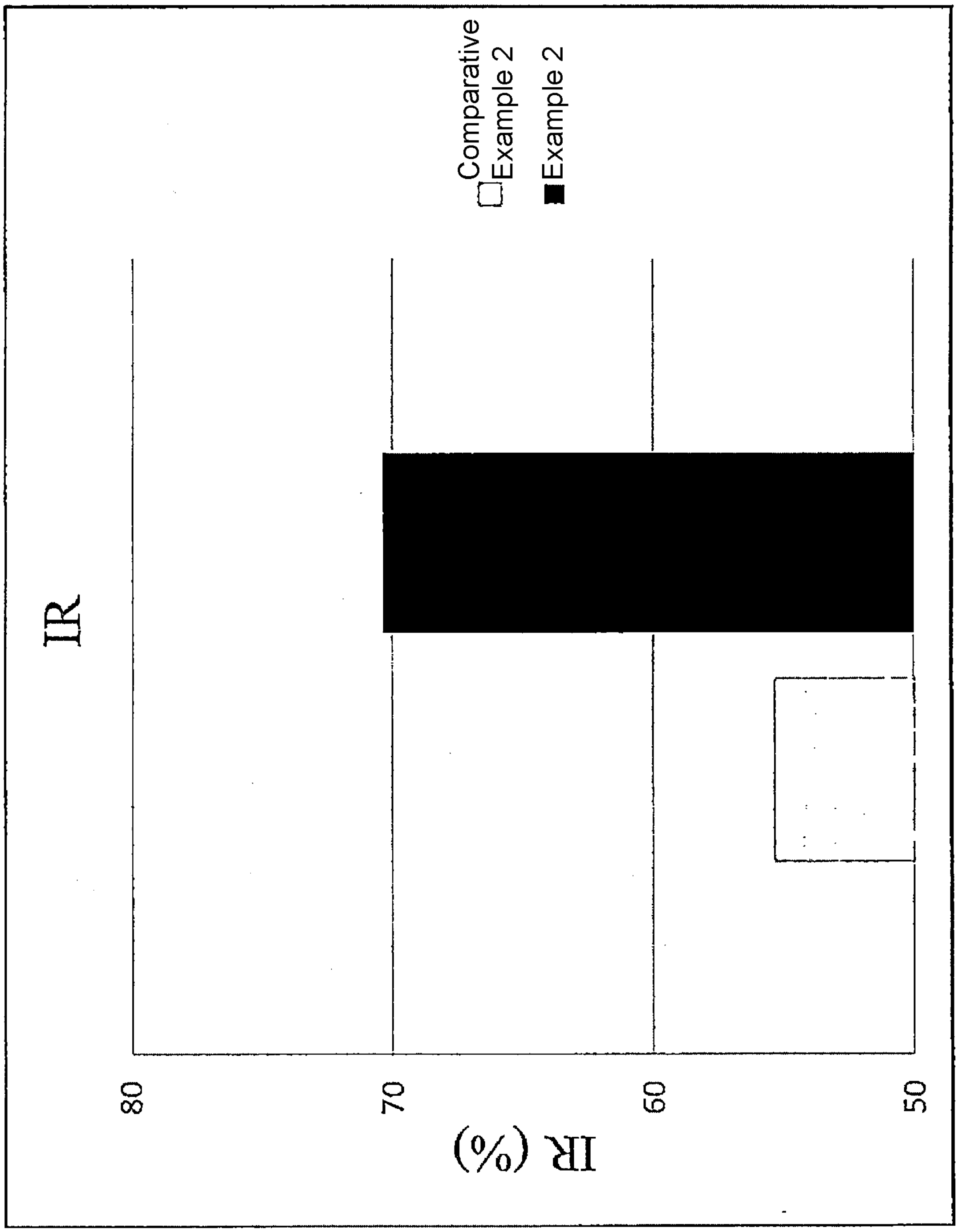

Fig. 2

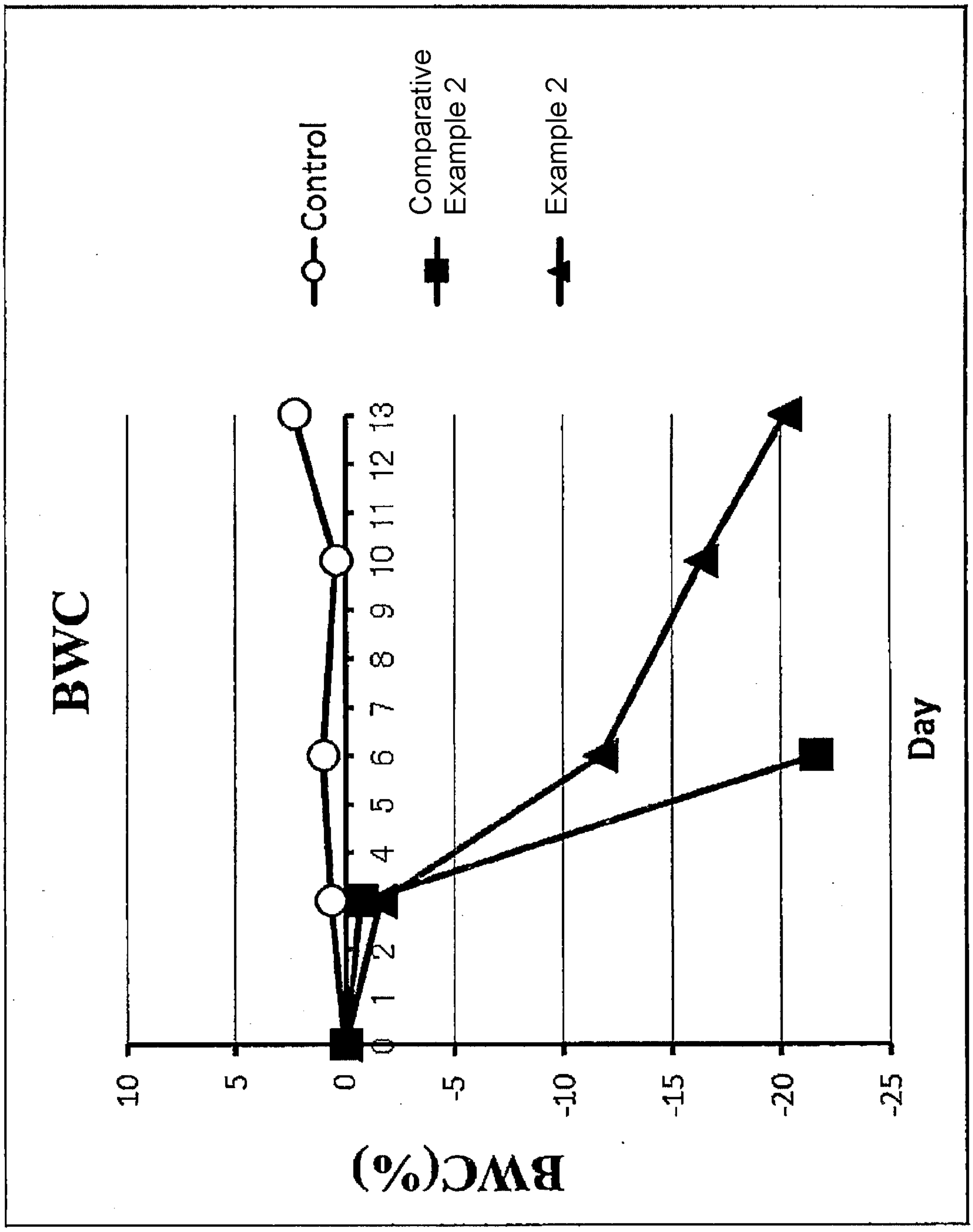
BWC
BWC(%)
10
5
0
-5
-10
-15
-20
-25
0 1 2 3 4 5 6 7 8 9 10 11 12 13
Day
Control
Comparative Example 2
Example 2

Fig. 3

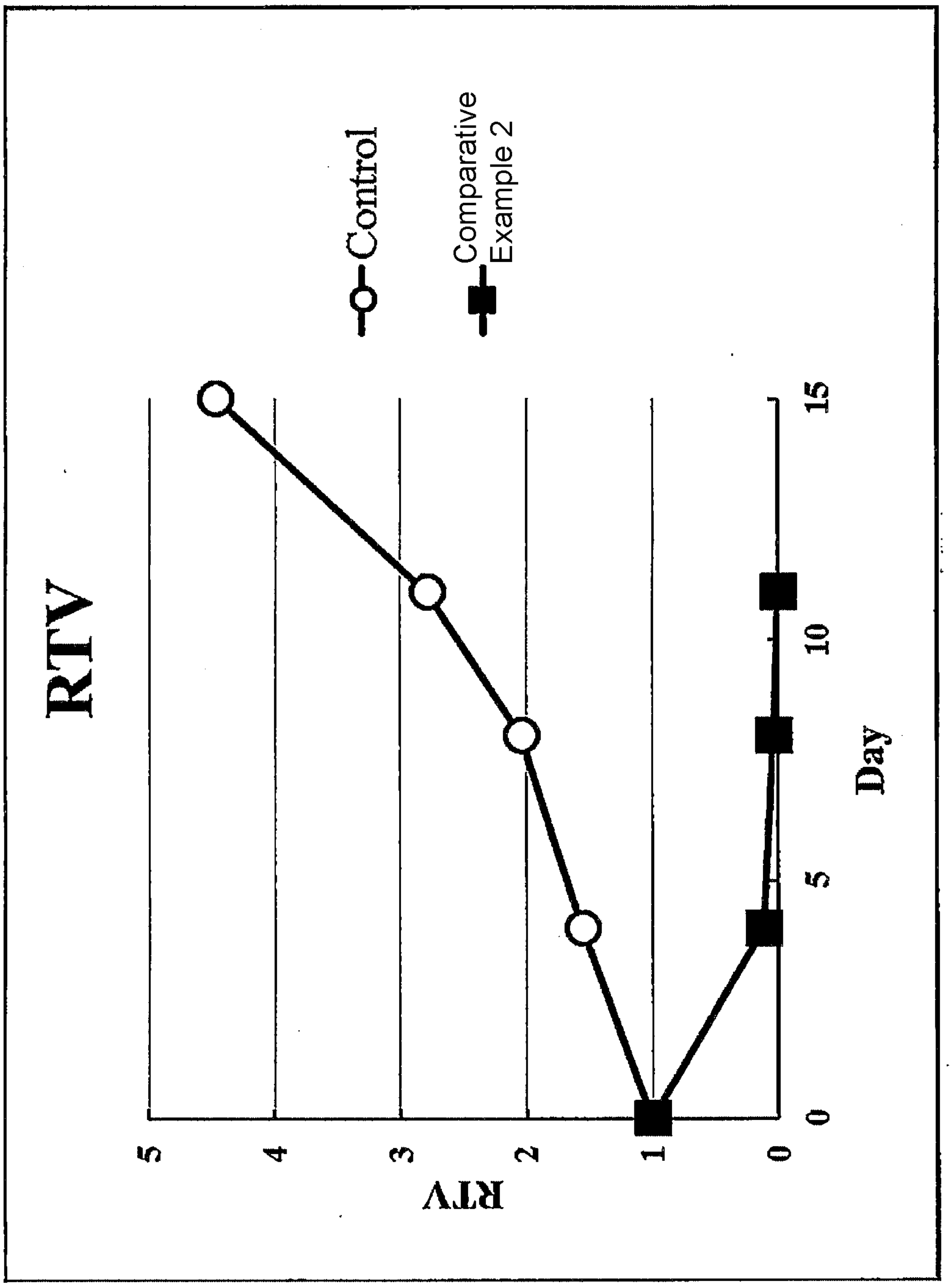

Fig. 4

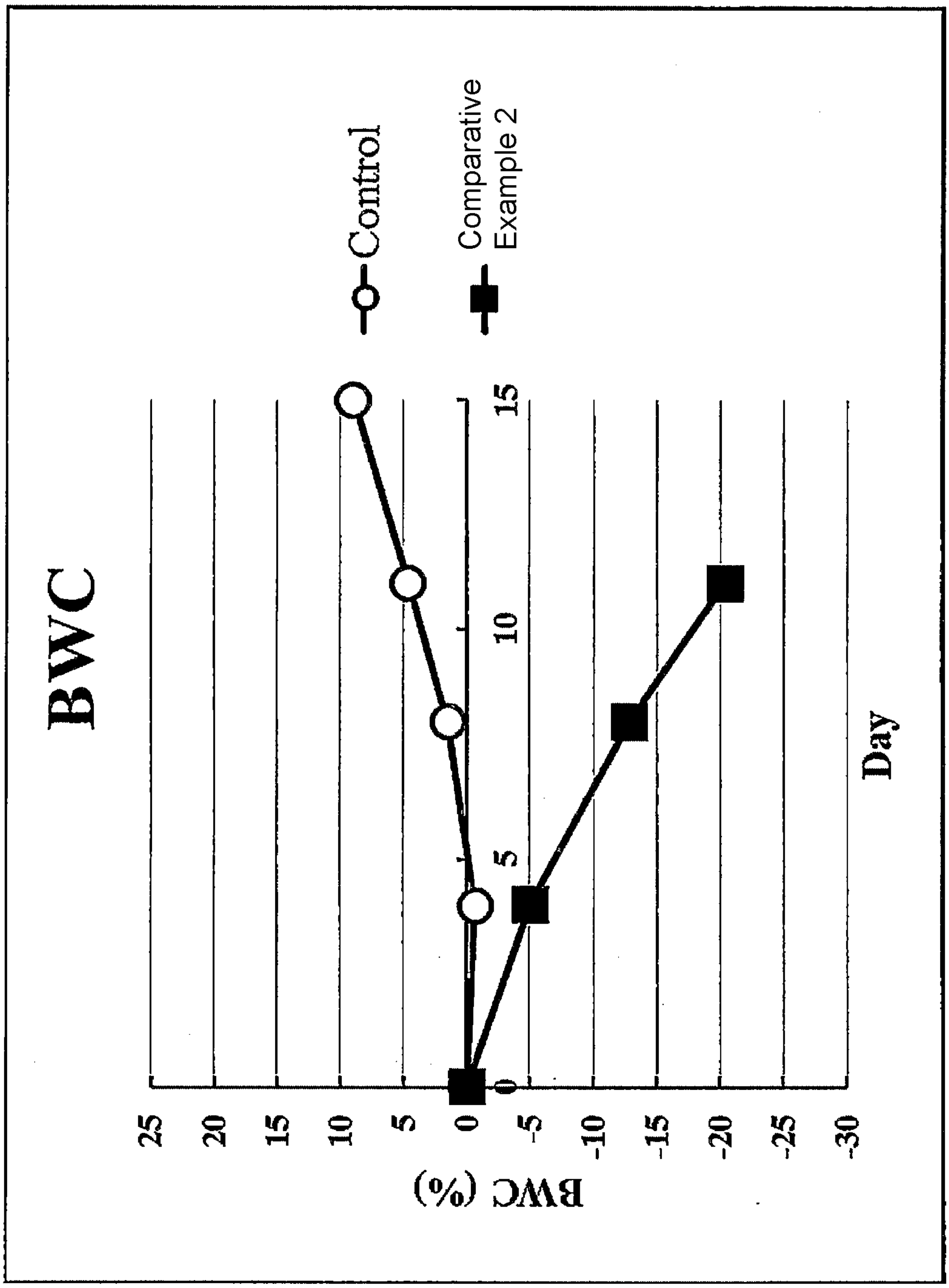
BWC
Control
Comparative Example 2
BWC (%)
Day
25
20
15
10
5
0
-5
-10
-15
-20
-25
-30
0
5
10
15

Fig. 5

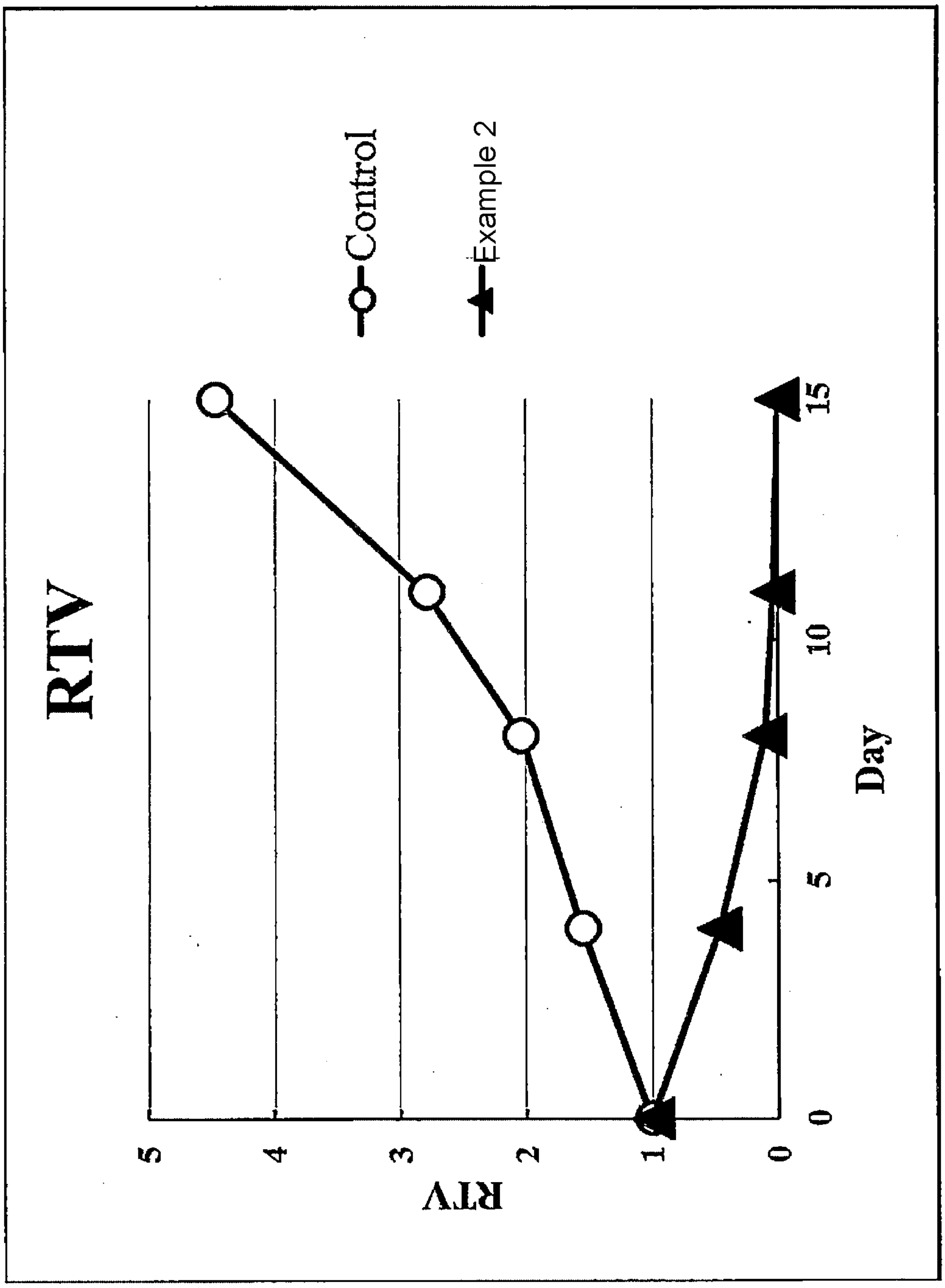

Fig. 6

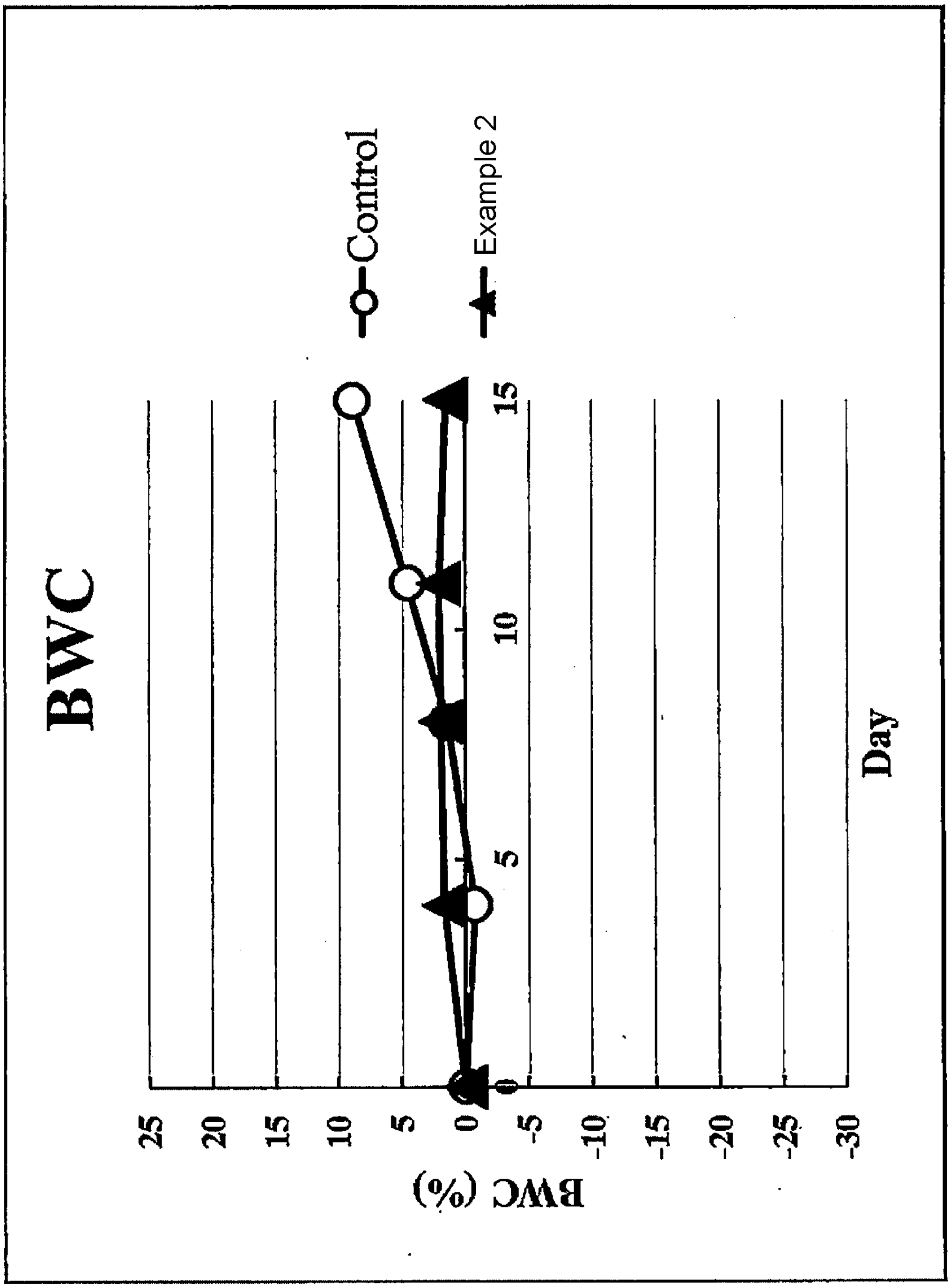
BWC
Control
Example 2
BWC (%)
Day
25
20
15
10
5
0
-5
-10
-15
-20
-25
-30
0
5
10
15

Fig. 7

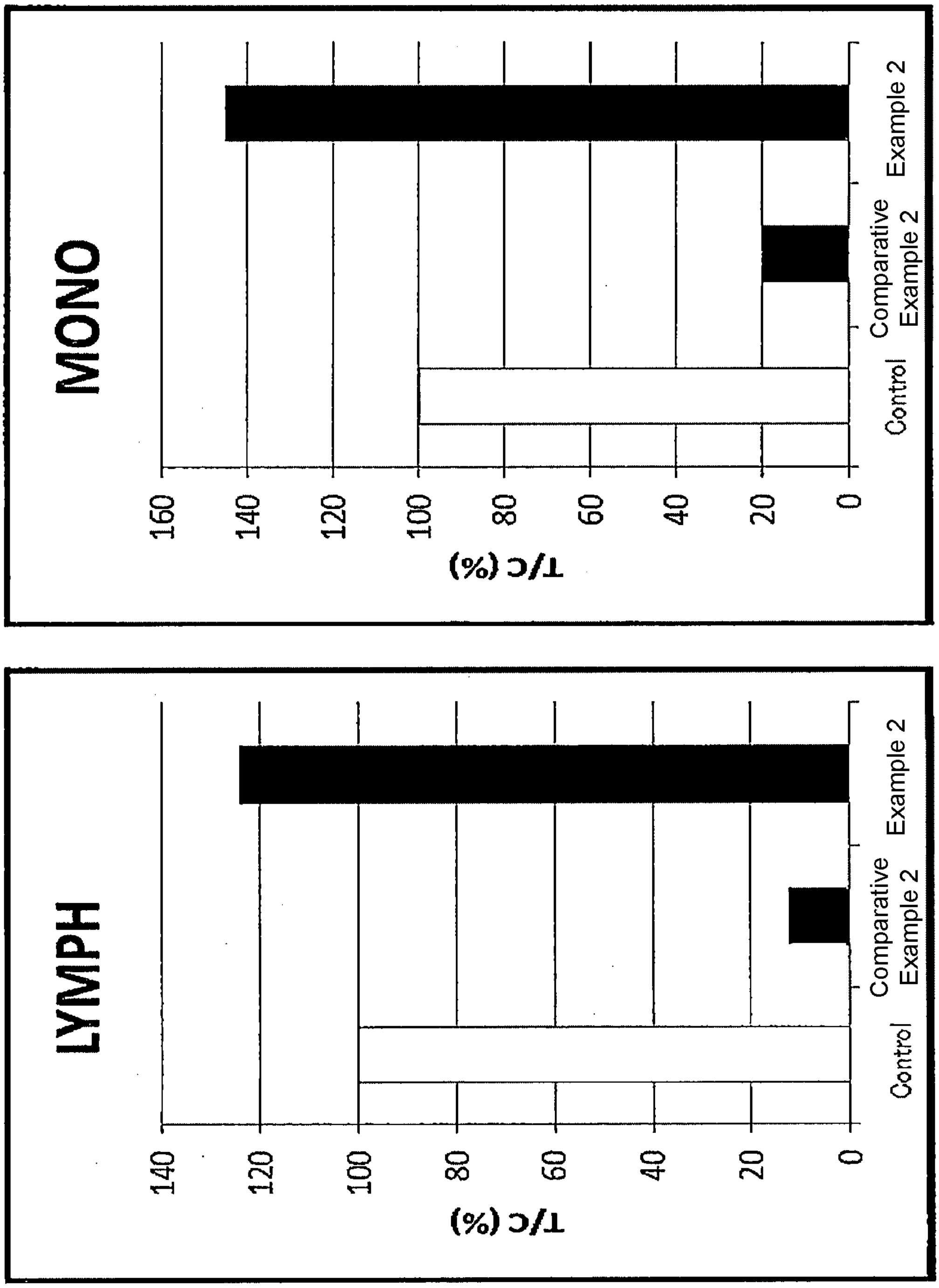
MONO
T/C (%)
160
140
120
100
80
60
40
20
0
Control
Comparative Example 2
Example 2
LYMPH
T/C (%)
140
120
100
80
60
40
20
0
Control
Comparative Example 2
Example 2

Fig. 8

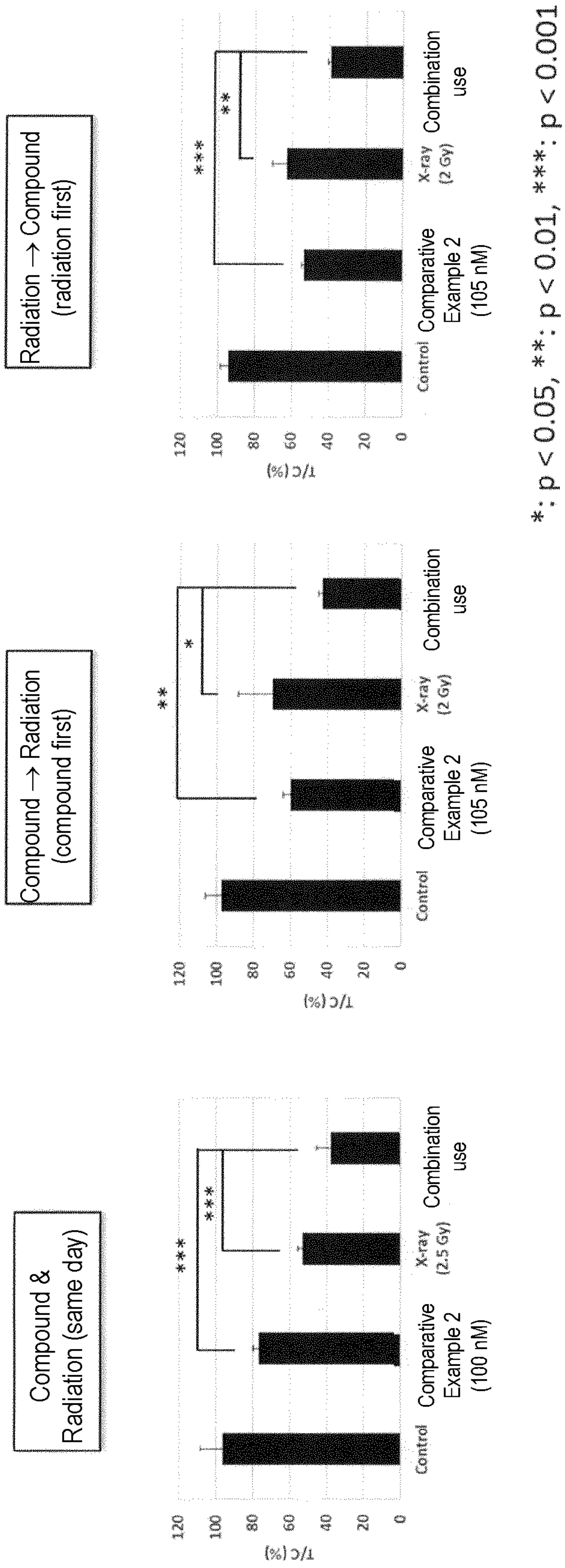
Radiation → Compound (radiation first)
Compound → Radiation (compound first)
Compound & Radiation (same day)
T/C (%)
120
100
80
60
40
20
0
Control
Comparative Example 2 (105 nM)
Comparative Example 2 (100 nM)
X-ray (2 Gy)
X-ray (2.5 Gy)
Combination use
*
**
***
*: p < 0.05, **: p < 0.01, ***: p < 0.001

Fig. 9

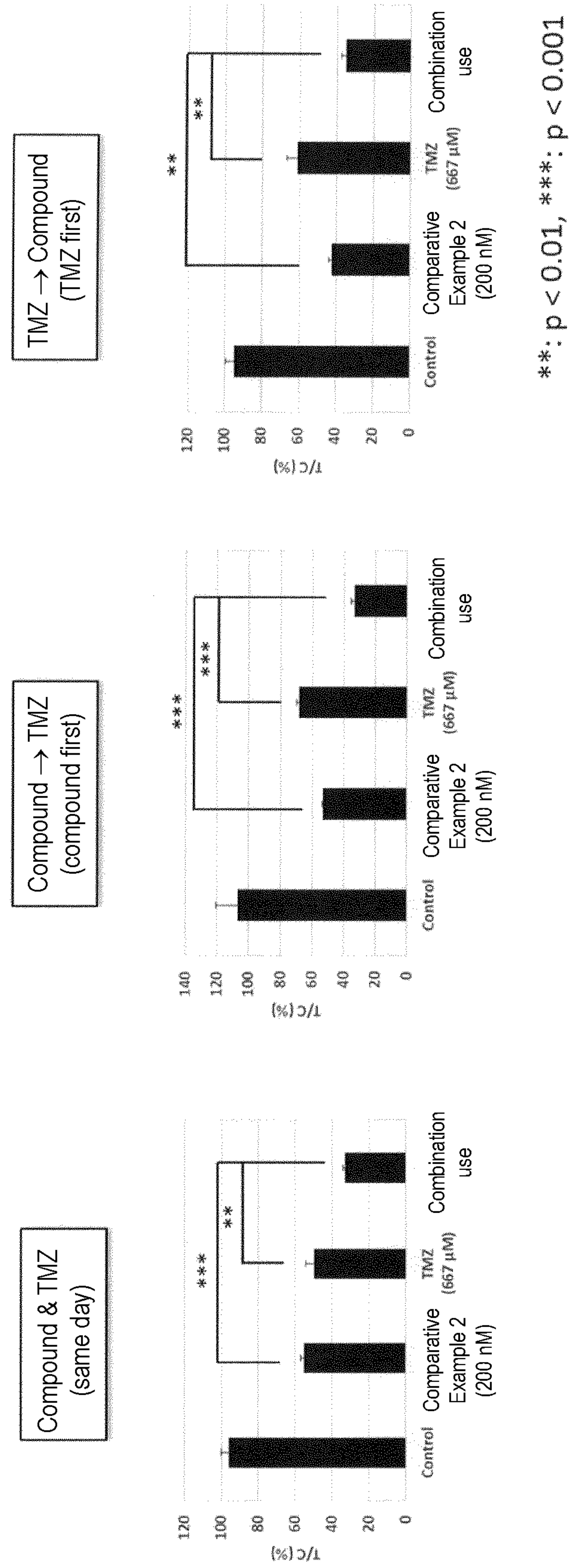
TMZ → Compound
(TMZ first)
Compound → TMZ
(compound first)
Compound & TMZ
(same day)
T/C (%)
Control
Comparative Example 2 (200 nM)
TMZ (667 μM)
Combination use
**
***
**: p < 0.01, ***: p < 0.001

## INTERNATIONAL SEARCH REPORT

International application No.
PCT/JP2020/023660

A. CLASSIFICATION OF SUBJECT MATTER
A61P 35/00(2006.01)i; C07H 19/14(2006.01)i; A61K 31/7064(2006.01)i
FI: C07H19/14 CSP; A61P35/00; A61K31/7064
According to International Patent Classification (IPC) or to both national classification and IPC

B. FIELDS SEARCHED
Minimum documentation searched (classification system followed by classification symbols)
A61P35/00; C07H19/14; A61K31/7064

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | Synthesis, 2006, (12), 2005-2012, DOI 10.1055/s-2006-942400 in particular, compound 1b | 21<br>1-61 |
| X<br>Y | Acta Crystallographica, Section C: Crystal Structure Communications, 2006, C62(4), 0231-0233, DOI 10.1107/S0108270106007633 in particular, compound I | 21<br>1-61 |
| X<br>Y | Acta Crystallographica, Section E: Structure Reports Online, 2014, 70(2), 0120, sup-1 to 6, DOI 10.1107/S1600536813034995 in particular, Structure Report and Related literature | 21<br>1-61 |
| X<br>Y | Organic & Biomolecular Chemistry, 2008, 6(19), 3552-3560, DOI 10.1039/b806145a in particular, compound 9 | 21<br>1-61 |

☒ Further documents are listed in the continuation of Box C.    ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 August 2020 (24.08.2020) | 08 September 2020 (08.09.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2020/023660

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | Bioorganic & Medicinal Chemistry, 2012, 20(17), 5202-5214, DOI 10.1016/j.bmc.2012.07.003 in particular, compound 11 | 21<br>1-61 |
| X<br>Y | Nucleosides, Nucleotides & Nucleic Acids, 2005, 24(5-7), 847-850, DOI 10.1081/NCN-200059181 in particular, compound 3b | 21<br>1-61 |
| X<br>Y | WO 2014/124430 A1 (EMORY UNIVERSITY) 14.08.2014 (2014-08-14) in particular, page 129 EFNS-01580 | 21<br>1-61 |
| Y<br>A | LARSEN, C. S. et al., Chapter 14 Design and application of prodrugs, Textbook of Drug Design and Discovery, Third edition, 2002, 460-514 in particular, page 479 | 1-20, 30-61<br>21-29 |
| Y<br>A | JP 2012-517443 A (RFS PHARMA, LLC) 02.08.2012 (2012-08-02) in particular, Title of invention, claims | 1-20, 30-61<br>21-29 |
| Y<br>A | JP 2012-524731 A (INSTITUTE OF ORGANIC CHEMISTRY AND BIOCHEMISTRY ASCR, V.V.I.) 18.10.2012 (2012-10-18) in particular, Title of invention, claims | 1-20, 30-61<br>21-29 |
| Y<br>A | Nucleosides, Nucleotides & Nucleic Acids, 2005, 24(10-12), 1635-1649, DOI 10.1080/15257770500267006 in particular, page 1635, title | 1-20, 30-61<br>21-29 |
| Y<br>A | Methods in Molecular Biology, 2004, 252(Ribozymes and siRNA Protocols), 57-75 in particular, page 67 fig. 3 | 1-20, 30-61<br>21-29 |
| Y<br>A | JP 2000-510127 A (DEUTSCHES KREBSFORSCHUNGSZENTRUM STIFTUNG DES ÖFFENTLICHEN RECHTS0 08.08.2000 (2000-08-08) in particular, Title of invention, claims | 1-20, 30-61<br>21-29 |

Form PCT/ISA/210 (continuation of second sheet) (January 2015)

**INTERNATIONAL SEARCH REPORT**

International application No.
PCT/JP2020/023660

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☒ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (January 2015)

**INTERNATIONAL SEARCH REPORT**
Information on patent family members

International application No.
PCT/JP2020/023660

| Patent Documents referred in the Report | Publication Date | Patent Family | Publication Date |
|---|---|---|---|
| WO 2014/124430 A1 | 14 Aug. 2014 | (Family: none) | |
| JP 2012-517443 A | 02 Aug. 2012 | US 2012/0040924 A1<br>in particular, Title of invention, claims<br>WO 2010/091386 A2<br>EP 2393815 A2<br>KR 10-2011-0115601 A<br>CN 102395590 A | |
| JP 2012-524731 A | 18 Oct. 2012 | US 2015/0218201 A1<br>in particular, Title of invention, claims<br>WO 2010/121576 A2<br>EP 2421879 A2<br>CN 102459300 A | |
| JP 2000-510127 A | 08 Aug. 2000 | WO 1997/043299 A1<br>in particular, Title of invention, claims<br>EP 918784 A1 | |

Form PCT/ISA/210 (patent family annex) (January 2015)

**INTERNATIONAL SEARCH REPORT**

International application No.
PCT/JP2020/023660

<Continuation of Box No. III>

Document 1: Synthesis, 2006, (12), 2005-2012, DOI 10.1055/s-2006-942400
Document 2: Acta Crystallographica, Section C: Crystal Structure Communications, 2006, C62(4), 0231-0233, DOI 10.1107/S0108270106007633
Document 3: Acta Crystallographica, Section E: Structure Reports Online, 2014, 70(2), 0120, sup-1 to 6, DOI 10.1107/S1600536813034995

(Invention 1) Claims 1-20 and parts of claims 30-61 dependent on claims 1-7

Claims 1-20 have the special technical feature of a "compound represented by general formula (1) or a pharmaceutically acceptable salt thereof", and are thus classified as invention 1.

In addition, the parts of claims 30-61 which are dependent on claims 1-7 and pertain to the "compound represented by general formula (1) or a pharmaceutically acceptable salt thereof" are classified as invention 1.

(Invention 2) Claims 21-29 and parts of claims 30-61 dependent on claim 21

Claims 21-29 pertain to a "compound represented by general formula (2) or a pharmaceutically acceptable salt thereof", are common to claim 1 classified as invention 1 in terms of a predetermined nucleoside structure up to oxygen atoms at a 5'-position and a 3'-position, and can be said to share the common technical feature.

However, said technical feature does not make a contribution over the prior art in light of the disclosures of documents 1-3 and thus cannot be said to be a special technical feature. Moreover, there do not exist other identical or corresponding special technical features between these inventions.

Furthermore, claims 21-29 are not dependent on claim 1. Moreover, claims 21-29 are not substantially identical or equivalent to any of the claims classified as invention 1.

Therefore, claims 21-29 cannot be classified as invention 1.

In addition, the parts of claims 30-61 which are dependent on claim 21 and pertain to the "compound represented by general formula (2) or a pharmaceutically acceptable salt thereof" are classified as invention 2.

Accordingly, the inventions are classified into two as follows.

(Invention 1) Claims 1-20 and parts of claims 30-61 dependent on claims 1-7
(Invention 2) Claims 21-29 and parts of claims 30-61 dependent on claim 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013009735 A
- WO 2014124430 A
- WO 2017165489 A


### Non-patent literature cited in the description


- **LI et al.** *Acta Cryst,* 2014, vol. E70, o120
- **PERLIKOVA et al.** *ChemMedChem,* 2013, vol. 8, 832-846
- **NAUS et al.** *Bioorg. Med. Chem,* 2012, vol. 20, 5202-5214
- **BROWN et al.** *J Med Chem.,* 28 July 2016, vol. 59 (14), 6860-6877
- **IKEHARA et al.** *Chemical & Pharmaceutical Bulletin,* 1966, vol. 14 (12), 1338-46
- **GREENE ; WUTS.** Protective Groups in Organic Synthesis. John Wiley & Sons Inc, 2014
- *Bioorg. Med. Chem,* 2012, vol. 20, 5202-5214
- *ChemMedChem,* 2013, vol. 8, 832-846